(19) Europäisches Patentamt / European Patent Office / Office européen des brevets

(11) **EP 4 324 559 B1**

(12) **EUROPEAN PATENT SPECIFICATION**

(45) Date of publication and mention of the grant of the patent:
28.01.2026 Bulletin 2026/05

(21) Application number: 21937015.2

(22) Date of filing: 16.04.2021

(51) International Patent Classification (IPC):
*B01J 23/42* (2006.01)   *B01J 21/04* (2006.01)
*C07C 5/367* (2006.01)   *C07C 15/06* (2006.01)
*C07B 61/00* (2006.01)   *B01J 35/30* (2024.01)
*B01J 37/18* (2006.01)   *B01J 37/20* (2006.01)
*C01B 3/26* (2006.01)

(52) Cooperative Patent Classification (CPC):
(C-Sets available)
C07C 5/367; B01J 21/04; B01J 23/42;
B01J 35/393; B01J 35/397; B01J 35/399;
B01J 35/45; B01J 35/53; B01J 35/615;
B01J 35/635; B01J 35/647; B01J 35/66;
B01J 37/18; B01J 37/20; C01B 3/26;   (Cont.)

(86) International application number:
PCT/JP2021/015767

(87) International publication number:
WO 2022/219821 (20.10.2022 Gazette 2022/42)

(54) **PLATINUM-SUPPORTING ALUMINA CATALYST, METHOD FOR PRODUCING SAME, AND METHOD FOR DEHYDROGENATING HYDROGENATED AROMATIC COMPOUNDS USING PLATINUM-SUPPORTING ALUMINA CATALYST**

PLATINUMTRAGENDER ALUMINIUMOXIDKATALYSATOR, VERFAHREN ZUR HERSTELLUNG DAVON UND VERFAHREN ZUR DEHYDRIERUNG VON HYDRIERTEN AROMATISCHEN VERBINDUNGEN MIT PLATINUMTRAGENDEM ALUMINIUMOXIDKATALYSATOR

CATALYSEUR D'ALUMINE SUPPORTANT LE PLATINE, SON PROCÉDÉ DE PRODUCTION ET PROCÉDÉ DE DÉSHYDROGÉNATION DE COMPOSÉS AROMATIQUES HYDROGÉNÉS À L'AIDE D'UN CATALYSEUR D'ALUMINE SUPPORTANT LE PLATINE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(43) Date of publication of application:
21.02.2024 Bulletin 2024/08

(73) Proprietor: Chiyoda Corporation
Kanagawa 220-8765 (JP)

(72) Inventors:
• OKADA, Yoshimi
Yokohama-shi, Kanagawa 220-8765 (JP)
• IMAGAWA, Kenichi
Yokohama-shi, Kanagawa 220-8765 (JP)
• NAKATA, Shinichi
Yokohama-shi, Kanagawa 220-8765 (JP)

(74) Representative: Dehns
10 Old Bailey
London EC4M 7NG (GB)

(56) References cited:
EP-A1- 1 894 626       WO-A1-2006/137358
JP-A- 2005 211 845     JP-A- 2011 143 408
JP-A- 2018 144 016     JP-B2- 4 652 695
JP-B2- 6 792 550

- OKADA ET AL: "Development of dehydrogenation catalyst for hydrogen generation in organic chemical hydride method", INTERNATIONAL JOURNAL OF HYDROGEN ENERGY, ELSEVIER, AMSTERDAM, NL, vol. 31, no. 10, 1 August 2006 (2006-08-01), pages 1348 - 1356, XP005498655, ISSN: 0360-3199, DOI: 10.1016/J.IJHYDENE.2005.11.014
- KWAK YEONSU ET AL: "Effect of the support properties in dehydrogenation of biphenyl-based eutectic mixture as liquid organic hydrogen carrier (LOHC) over Pt/Al2O3 catalysts", FUEL, IPC SIENCE AND TECHNOLOGY PRESS , GUILDFORD, GB, vol. 284, 9 October 2020 (2020-10-09), XP086335026, ISSN: 0016-2361, [retrieved on 20201009], DOI: 10.1016/ J.FUEL.2020.119285

(52) Cooperative Patent Classification (CPC): (Cont.)
**C07B 61/00;** B01J 2235/30; C07C 2521/04; Y02P 20/52

C-Sets
**C07C 5/367, C07C 15/06**

**Description**

TECHNICAL FIELD

The present invention relates to a metal catalyst for use in processes using catalyst, such as chemical product production, hydrogen production, fine chemical production, environment cleaning such as exhaust gas treatment, etc., and relates to a platinum-loaded alumina catalyst in which platinum is loaded on an alumina carrier, a method of producing the same, and a method of dehydrogenating a hydrogenated aromatic using the catalyst.

BACKGROUND ART

A platinum-loaded alumina catalyst in which platinum or the like is loaded on an alumina carrier is industrially used in a very wide range of fields, such as production of fuel, petrochemical products, and fine chemicals such as medicine or environmental clean-up such as cleaning automobile exhaust gas, through dehydrogenation reaction, hydrogenation reaction, and reforming reaction of various compounds, such as dehydrogenation reaction of dehydrogenating hydrogenated aromatics such as methylcyclohexane, cyclohexane, decalin, and dibenzyltoluene, for example, into the corresponding aromatics and hydrogen.

Generally, such platinum-loaded alumina catalyst is manufactured as follows: a porous alumina carrier made of a metal oxide of alumina is prepared; the obtained porous alumina carrier is impregnated with a solution of a catalyst metal compound, such as a chloroplatinic acid aqueous solution, a platinum ammonium chloride aqueous solution, and a solution of an organoplatinum compound such as platinum acetylacetonate; the resultant is dried to form a dried matter on which the catalyst metal compound is loaded; the dried matter is calcined, e.g., at 350 to 800 °C for 0.5 to 24 hours to form a calcined matter on which the catalyst metal compound is loaded; and, as required, the obtained calcined matter on which the catalyst metal compound is loaded is subjected to hydrogen reduction, e.g., at 250 to 800 °C for 0.5 to 24 hours.

Regarding the platinum-loaded alumina catalyst manufactured by such a method, it is known that since the platinum atom with the atomic weight 195 has a large mass and the adsorbability of a platinum compound used as a platinum source to the catalyst carrier is high, the platinum compound is adsorbed by and fixed to the outer shell part of the alumina carrier before the platinum compound is dispersed to the inside of the alumina carrier, which forms a so-called egg shell-type platinum-loaded catalyst in which, when the dispersion state of platinum metal is observed in the catalyst cross section, the platinum metal is loaded only on the outer shell part of the catalyst and no platinum metal is loaded inside the carrier.

In the catalytic reaction in which the diffusion resistance inside the catalyst particles is high due to reasons such as that raw material molecules are large, the reaction occurs preferentially in the outer shell of the catalyst particles even if platinum as the active metal is loaded to the inside of the catalyst because the speed of diffusion of the raw material molecules to the inside of the catalyst is slow and the reaction does not progress sufficiently. In such a reaction, the egg shell-type catalyst in which the active metal exists in only the outer shell part of the catalyst is advantageous. However, when a certain amount of active metal is loaded only on the outer shell of the catalyst particles, the density of the active metal particles increases, which may lead to problems that the active metal particles cannot be sufficiently dispersed, catalyst deterioration due to sintering or coking is likely to occur, etc. From such a viewpoint, catalysts in which the dispersity of platinum metal when platinum metal is loaded only on the outer shell part of the catalyst is improved are being developed. Patent Document 1 discloses an egg shell-type platinum-loaded alumina catalyst in which the pore size of the platinum-loaded alumina catalyst is substantially uniform to such a degree that the diffusion resistance does not become large and the dispersion of platinum is good. Also, catalysts in which platinum metal is dispersed well over the entire cross section of the catalyst so that the surface area of the carrier is fully utilized in a reaction not affected by the diffusion resistance are being developed, and Patent Document 2 discloses such a uniform-type platinum-loaded alumina catalyst.

The platinum-loaded alumina catalyst has been used in the catalytic process of a wide range of fields from old times, but in recent years is used in an organic chemical hydride method which is one method for hydrogen energy carrier and is attracting attention as storage and transportation technology for hydrogen energy. Development of platinum-loaded alumina catalyst having higher performance compared to the conventional platinum-loaded alumina catalyst is being in progress, and Patent Document 1 and Patent Document 2 disclose, as a use of the platinum-loaded alumina catalyst, use in the dehydrogenation reaction necessary in the organic chemical hydride method. The organic chemical hydride method is a method for "storing" and "transporting" hydrogen in the form of organic chemical hydride compounds (hydrogenated organic compounds) in which hydrogen is taken in the molecular structure of chemical products by chemical reaction. The organic chemical hydride method is a method that has been proposed since 1980s. However, the life of dehydrogenation catalyst for producing hydrogen from the organic chemical hydride compounds in which hydrogen is retained is very short, whereby industrial implementation thereof is difficult and the method has not been put into practice. The key to technological development is development of a novel dehydrogenation catalyst having sufficient performance such as a catalyst life that allows for industrial use. Currently, due to application of a platinum-loaded alumina catalyst having high performance as mentioned above, the development of a hydrogen energy carrier system based on the organic chemical hydride method has reached a point where demonstration of large-scale international hydrogen transportation is executed, and the development has technologically progressed to a stage where commercialization is possible. Non-Patent Documents 3 and 4 disclose details of such development of the organic chemical hydride method.

Japan has adopted a policy to promote practical implementation and dissemination of hydrogen energy as a national policy from the Fourth Strategic Energy Plan after the Great East Japan Earthquake, and following formulation of the Hydrogen Fuel Cell Technology Roadmap, the Hydrogen Basic Strategy was approved by the cabinet in 2017. The aforementioned organic chemical hydride method can provide a hydrogen energy carrier for "storing" and "transporting" hydrogen energy in a large scale, and the practical implementation thereof is incorporated in the Hydrogen Basic Strategy, which sets 30 ¥/Nm$^3$ as a target hydrogen supply price by 2030, and 20 ¥/Nm$^3$ by 2050. This requires cost reduction by continuous improvement technology development, and improvement of the catalyst performance, particularly the catalyst life, significantly affects the cost reduction. The development has progressed such that of the catalyst performance, the conversion rate, the selectivity, and the yield, which is a product of the conversion rate and the selectivity, have been improved to a relatively high level, and the development is now in a stage where improvement of the catalyst life, which determines how long the performance can be maintained, contributes to the cost reduction.

PRIOR ART DOCUMENT(S)

PATENT DOCUMENT(S)

Patent Document 1: JP4652695B2
Patent Document 2: JP4142733B2
Non-Patent Document 3: OKADA Yoshimi, Energy/Natural Resources, Vol.33, No.3, 168 (2018)
Non-Patent Document 4: OKADA Yoshimi, Bulletin of The High Pressure Gas Safety Institute of TOKYO, August and September 2019
Non-Patent Document 5: Agency for Natural Resources and Energy, Hydrogen Basic Strategy (December 2017)

SUMMARY OF THE INVENTION

TASK TO BE ACCOMPLISHED BY THE INVENTION

Under the above background, the present inventors carried out extensive research on the preparation method in loading platinum on an alumina carrier or a sulfur-containing alumina carrier for the purpose of improving the catalyst life of the egg shell-type platinum-loaded alumina catalyst and the uniform-type platinum-loaded alumina catalyst respectively disclosed in Patent Documents 1 and 2.

As a result, the inventors found that regarding the egg shell-type platinum-loaded alumina catalyst and the uniform-type platinum-loaded alumina catalyst, the catalyst life can be remarkably improved compared to the conventional platinum-loaded alumina catalyst by properly setting the particle diameters of the platinum particles loaded on the alumina carrier (by properly controlling the platinum particle distribution).

Thus, an object of the present invention is to provide a platinum-loaded alumina catalyst with an improved catalyst life, a manufacturing method thereof, and a method of dehydrogenating a hydrogenated aromatic using the catalyst.

MEANS TO ACCOMPLISH THE TASK

One aspect of the present invention is a platinum-loaded alumina catalyst, comprising: an alumina carrier; and platinum loaded on the alumina carrier, wherein the alumina carrier comprises a γ-alumina carrier that has a surface area of 200 m$^2$/g or more, a pore volume of 0.50 cm$^3$/g or more, and an average pore diameter in a range of 6.0 to 15.0 nm (60 to 150 Å), with pores having a pore diameter in a range of ±3.0 nm (± 30 Å) from the average pore diameter occupying 60 % or more of a total pore volume, particles of the platinum are loaded on the γ-alumina carrier in a range of 0.1 to 1.5 % by weight calculated as elemental platinum (Pt), and 70 % or more of the particles of the platinum have a size of 0.8 to 1.5 nm (8 to 15 Å) by direct observation using a transmission electron microscope.

According to this aspect, it is possible to improve the catalyst life of the platinum-loaded alumina catalyst by properly setting the particle diameters of the platinum particles (by properly controlling the platinum particle distribution). Also, according to this aspect, since the pore distribution of the γ-alumina carrier is sharply controlled, the pore size is uniform over the entirety of the powder and molded body thereof, and there is also an advantage that a process of dispersing and loading platinum and a process of dispersing and loading platinum over the entirety (for example, in correspondence with the distribution of sulfur) can be stably executed.

In the platinum-loaded alumina catalyst of the above aspect, preferably, the γ-alumina carrier contains sulfur or a sulfur compound in a range of 0.5 to 1.2 % by weight calculated as elemental sulfur (S).

In the platinum-loaded alumina catalyst of the above aspect, preferably, the γ-alumina carrier has alkali metals loaded thereon in a range of 0.5 to 1.5 % by weight, and the alkali metals are sodium and potassium.

Another aspect of the present invention is a method of producing the platinum-loaded alumina catalyst according to the above aspect, wherein in a preparation of the γ-alumina carrier, an alkaline aqueous solution is added to an acidic aqueous solution containing aluminum, and a boehmite obtained as aluminum hydroxide is dried and thereafter is calcined at a temperature in a range of 250 to 400 °C for a time period in a range of 1 to 12 hours.

In the method of producing a platinum-loaded alumina catalyst of the above aspect, preferably, by using a chloroplatinic acid aqueous solution as a platinum reagent aqueous solution, the γ-alumina carrier after the calcination is impregnated with the platinum such that a content thereof is in a range of 0.5 to 1.5 % by weight calculated as elemental platinum, and a resultant is dried and thereafter is calcined at a temperature in a range of 250 to 400 °C.

According to this aspect, by preparing the γ-alumina carrier in an appropriate condition, it is possible to properly set the particle diameters of the platinum particles (properly control the platinum particle distribution), and as a result, to improve the catalyst life of the platinum-loaded alumina catalyst produced. Also, according to this aspect, the platinum-loaded alumina catalyst can be mass-produced easily by existing catalyst production facilities.

In the method of producing a platinum-loaded alumina catalyst of the above aspect, preferably, the γ-alumina carrier that has been impregnated with the platinum, dried, and thereafter calcined is subjected to hydrogen reduction, and a temperature of the hydrogen reduction is higher than the temperature for calcining the boehmite after drying, is higher than the temperature for calcining the γ-alumina carrier impregnated with the platinum and dried, and is in a range of 300 to 450 °C.

In the method of producing a platinum-loaded alumina catalyst of the above aspect, preferably, a time period of the hydrogen reduction is in a range of 1 to 15 hours.

In the method of producing a platinum-loaded alumina catalyst of the above aspect, preferably, the γ-alumina carrier contains sulfur or a sulfur compound in a range of 0.5 to 1.2 % by weight calculated as elemental sulfur (S).

In the method of producing a platinum-loaded alumina catalyst of the above aspect, preferably, the γ-alumina carrier after the calcination is impregnated with ammonium sulfate aqueous solution and thereafter is calcined at a temperature in a range of 250 to 400 °C for a time period in range of 1 to 12 hours.

In the method of producing a platinum-loaded alumina catalyst of the above aspect, preferably, the γ-alumina carrier containing no sulfur or the γ-alumina carrier containing sulfur is impregnated with platinum, is dried, and thereafter is calcined to generate a platinum-loaded γ-alumina carrier, the platinum-loaded γ-alumina carrier is impregnated with alkali metals such that the alkali metals are contained in a range of 0.5 to 1.5 % by weight, is dried, and thereafter is subjected to hydrogen reduction without being calcined, and a temperature of the hydrogen reduction is higher than the temperature for calcining the boehmite after drying, is higher than the temperature for calcining the γ-alumina carrier impregnated with the platinum and dried, and is in a range of 300 to 450 °C.

In the method of producing a platinum-loaded alumina catalyst of the above aspect, preferably, the alkali metals are sodium and potassium.

In the method of producing a platinum-loaded alumina catalyst of the above aspect, preferably, a time period of the hydrogen reduction is in a range of 1 to 15 hours.

Another aspect of the present invention is a method of dehydrogenating a hydrogenated aromatic, comprising dehydrogenating a hydrogenated aromatic by using the platinum-loaded alumina catalyst according to the above aspect.

According to this aspect, with the platinum-loaded alumina catalyst having an improved catalyst life, the dehydrogenation of the hydrogenated aromatics can be stably executed.

In the method of dehydrogenating a hydrogenated aromatic of the above aspect, preferably, the hydrogenated aromatic is one member or a mixture of two or more members selected from the group consisting of a hydride of monocyclic aromatic, a hydride of bicyclic aromatic, and a hydride of compound having 3 or more aromatic rings.

In the method of dehydrogenating a hydrogenated aromatic of the above aspect, preferably, the hydrogenated aromatic is one member or a mixture of two or more members selected from the group consisting of methylcyclohexane, cyclohexane, trimethylcyclohexane, decalin, and dibenzotriol.

Regarding the platinum-loaded alumina catalyst according to the present invention, the uniform-type catalyst is effective when the raw material is sufficiently diffused to the inside of the catalyst, and the egg shell-type catalyst is effective when the diffusion to the inside of the catalyst is limited and is not performed sufficiently, and therefore, it is possible to use these two types of catalysts properly depending on the state of diffusion in the reaction field. Also, even with the same reaction, the state of diffusion of the raw material to the inside of the catalyst may differ depending on the position in the reactor, and near the exit where the reaction has progressed, the raw material concentration becomes low, and the diffusion to the inside of the catalyst may be limited. In such a case, it is possible to use both of the uniform-type and egg shell-type catalysts in the reactor.

In general, the extent of diffusion of the raw material to the inside of the catalyst is represented by a catalyst effectiveness factor, and the catalyst effectiveness factor can be controlled by changing the size and shape of the catalyst pellet. Thus, for both of the uniform-type and egg shell-type catalysts, it is possible to produce platinum-alumina catalysts having various catalyst effectiveness factors by changing the size and shape of the catalyst pellet.

In the platinum-loaded alumina catalyst according to the present invention, it is preferred that the alumina carrier on which platinum is loaded has pore sizes controlled as uniformly as possible so that the pore distribution thereof is sharp. Specifically, a $\gamma$-alumina carrier having a surface area of 200 $m^2$/g or more, a pore volume of 0.5 $cm^3$/g or more, and an average pore diameter of 6.0 to 15.0 nm (60 to 150 Å), with pores having a pore diameter in a range of $\pm$ 3.0 nm ($\pm$ 30Å) from the average pore diameter occupying 60 % or more of a total pore volume is preferred. If the surface area is less than 200 $m^2$/g, the activity after the catalyst is formed is not sufficient, and if the pore volume is less than 0.5 $cm^3$/g, uniform loading of the active metal component is difficult, if the average pore diameter is less than 6.0 nm (60 Å), the pore volume becomes small while the surface area becomes large, and if, to the contrary, the average pore diameter is larger than 15.0 nm (150 Å), the surface area becomes small while the pore volume becomes large. When these correlations are taken into consideration comprehensively, an average pore diameter of 6.0 to 15.0 nm (60 to 150 Å) is appropriate. Also, if the pores having a pore diameter in a range $\pm$ 3.0 nm ($\pm$ 30 Å) from the average pore diameter occupy less than 60 %, the effect of the present invention in the catalyst performance becomes less. As a result of that the pore sizes are made uniform ass this, the alumina carrier has a uniform pore size over the entirety of the powder and molded body thereof. As a result, it is possible to properly execute a process of dispersing and loading platinum on the alumina carrier and a process of dispersing and loading platinum over the entirety in correspondence with the distribution of sulfur.

Patent Document 1 discloses a platinum-loaded alumina catalyst in which platinum is loaded on a porous $\gamma$-alumina carrier having a surface area of 150 $m^2$/g or more, a pore volume of 0.55 $cm^3$/g or more, and an average pore diameter of 9.0 to 30.0 nm (90 to 300 Å), with pores having a pore diameter of 9.0 to 30.0 nm (90 to 300 Å) occupying 60 % or more of a total pore volume. This catalyst is a general egg shell-type platinum-loaded alumina catalyst as described above, and Patent Document 1 also discloses a catalyst to which alkali metal is added as means to improve the catalyst life. When using a catalyst with alumina as a carrier, merely inhibiting the decomposition reaction that occurs on the platinum particles is not enough, and it is also necessary to inhibit the decomposition reaction that occurs on the acid sites of the alumina. Therefore, in many cases, the decomposition reaction occurring on the alumina surface is inhibited by masking these acid sites using alkali metals such as potassium and lithium. From this viewpoint, Patent Document 1 discloses that in the egg shell-type platinum-loaded catalyst, loading of platinum with high dispersity inhibits the decomposition on platinum, and addition of alkali to mask the acid sites on alumina has a remarkable effect on the catalyst life improvement.

On the other hand, Patent Document 2 discloses a uniform-type platinum-loaded alumina catalyst in which sulfur is contained in the alumina carrier so that the dispersion mode of the loaded platinum becomes a uniform-type and the decomposition reaction is suppressed to improve the catalyst life. At the time when the invention of Patent Document 2 was made, it was considered that in the case where the alumina carrier containing sulfur or a sulfur compound is used, even when the acid sites are not masked with alkali metal thereon, the decomposition reaction inhibitory effect is equivalent to or higher than the inhibitory effect when the acid sites are masked with alkali metal. Although the detailed mechanism is not elucidated, it was considered that elemental sulfur forms a complex oxide with alumina, thereby altering the configuration of the acid sites, which remain in the case of using alumina alone, to a different configuration. In that case, the form obtained when elemental sulfur forms a complex oxide with alumina is generally considered to be in a sulfate group form. The sulfate group itself is acidic and the number of acid sites, i.e., acidity, presumably increases by the existence thereof. There is an effect that these acid sites do not cause the decomposition reaction to proceed at a relatively low reaction temperature, but it is found that masking the remaining acid sites by adding alkali metals is effective.

In the catalyst production, additional inclusion of the alkali addition process can be a cause of cost increase, but the cost reduction effect brought by the extension of the catalyst life according to the present invention is much greater than the increase in the catalyst production cost. It has become possible to extend the replacement life of the dehydrogenation catalyst from the conventional 1 to 2 years to 3 to 4 years depending on how the catalyst is used in the reactor.

The platinum-loaded alumina catalyst according to the present invention is typically used as a dehydrogenation catalyst. Also, the platinum-loaded alumina catalyst is used by filling the catalytic reaction tubes of a heat exchange-type reactor. As with general heat exchanges, the number of the catalytic reaction tubes may be thousands in a large reactor. When the platinum-loaded alumina catalyst used in such a reactor reaches a catalyst life, at which time the performance is lowered to result in a certain yield, the catalyst is removed to be replaced with new catalyst. Platinum is recovered from the removed waste catalyst and is recycled for use in the production of the catalyst for next replacement. The removing work may take several days, and the charging of the new catalyst may require more working days, and therefore, the catalyst replacement requires about two weeks. The production stops during that time, and therefore, the reduction of replacement frequency significantly contributes to the cost reduction. Namely, while the life of the catalysts disclosed by Patent Document 1 and Patent Document 2 is 1 to 2 years, the life of the platinum-loaded alumina catalyst according to the present invention improves to about 4 years, and this makes it possible to reduce the catalyst replacement frequency to half or less. Due to the cost reduction effect brought by this increase of catalyst life, the overall economy could be improved even taking into account the increase of the catalyst production cost.

The egg shell-type platinum-loaded alumina catalyst and the uniform-type platinum-loaded alumina catalyst prepared by making sulfur contained in the γ-alumina carrier according to the present invention are used, for example, as a dehydrogenation catalyst for a hydrogenated aromatic, which is utilized as a hydrogen energy carrier in the organic chemical hydride method, which is one of the methods for storing and transporting the hydrogen energy. The hydrogenated aromatic preferably is one member or a mixture of two or more members selected from the group consisting of a hydride of monocyclic aromatic such as methylcyclohexane, cyclohexane, dimethylcyclohexane, and trimethylcyclohexane, a hydride of bicyclic aromatic such as tetralin, decalin, methyldecalin, biphenyl, and diphenylmethyl, and a hydride of compound having 3 or more aromatic rings such as dibenzotriol and tetradecahydroanthracene.

When the platinum-loaded alumina catalyst according to the present invention is used in the dehydrogenation reaction of a hydrogenated aromatic such as methylcyclohexane in the organic chemical hydride method, catalyst deterioration is observed, in which the performance of the catalyst decreases gradually as the reaction time passes. The cause of the catalyst deterioration is carbon precipitation called coking. In coking, carbon precipitation occurs on the surface of platinum metal, which is an active metal, mainly due to decomposition reaction of the raw material compound such as methylcyclohexane, and as a result, the effective active sites of the active metal are covered (namely, inactivation occurs due to decrease in the number of active sites) and the catalyst stops functioning.

The catalytic reaction deterioration phenomenon is observed as a decrease of the conversion rate in the reaction test, and in the case of dehydrogenation reaction of a hydrogenated aromatic, it is known that the phenomenon is observed as a linear decrease of the conversion rate. Accordingly, by observing the change of the conversion rate over time in the reaction test, it is possible to evaluate relative superiority or inferiority of the catalyst life based on the inclination of the decrease. Further, since the conversion rate decreases linearly not only under actual reaction conditions but also in an accelerated reaction test for evaluating the catalyst life in a short time, it is possible to evaluate the catalyst life.

Hydrogen attracted attention as clean secondary energy from 1970s, and in Japan, research and development of hydrogen production technologies and fuel cells were promoted in the Sunshine Project from 1974 to 1992, the Moonlight Project from 1978 to 1992, and the New Sunshine Project from 1993 to 2001. Regarding large-scale storage and transportation technology of hydrogen, development of liquefied hydrogen method was started in the WE-NET project from 1992 to 2002. On the other hand, the history of development of the organic chemical hydride method is old, and goes back to the Euro-Quebec project which was carried out in 1980s as an international research and development project by Canada's Quebec government and twelve European countries. This plan proposed producing hydrogen by performing water electrolysis by using excess hydroelectric power abundantly present in Quebec and transporting the hydrogen across the Atlantic Ocean for use in Europe. As a hydrogen transportation method, discussion was made on the liquid hydrogen method as a first candidate, the liquid ammonia method as a second candidate, and the organic chemical hydride method as a third candidate. At that time, the organic chemical hydride method was called an MCH method. The Euro-Quebec project continued till about 1992 for approximately 10 years but the project ended with none of the methods being practically implemented, and since then, technology for large-scale hydrogen store and transport has not been practically implemented.

In Japan, development of the liquefied hydrogen method was promoted in WE-NET project implemented from 1992 to 2002, while the research of the organic chemical hydride method was promoted mainly by Japanese universities. The applicant started the development of dehydrogenation catalyst in 2002 and made a first academic presentation in the World Hydrogen Energy Conference held in Yokohama in 2004, and from around this time, examples of research and development at companies were started to be released. At the present time, the large-scale hydrogen storage and transportation technologies for which research and development have been advanced to a demonstration level are only the liquefied hydrogen method and the organic chemical hydride method, which is proposed by the applicant.

The organic chemical hydride method (OCH method) is a method in which hydrogen is subjected to hydrogenation reaction with an aromatic such as toluene (TOL) and converted to a saturated cyclic compound such as methylcyclohexane (MCH) having the hydrogen taken in the molecule thereof so that "storage" and "transportation" are achieved in a liquid state under normal temperature and normal pressure and a necessary amount of hydrogen is taken out by dehydrogenation reaction and used at the place of use. Thus, the method includes a hydrogenation reaction (hydrogen storage reaction) for making hydrogen react with toluene and a dehydrogenation reaction (hydrogen generation reaction) for generating hydrogen from MCH and recovering toluene. TOL generated after taking out hydrogen is recovered and repeatedly used as a container (carrier) of hydrogen.

Since hydrogen is an explosive gas, there is a potentially high risk in storing and transporting hydrogen as it is in a large scale. In the present method, storage and transportation of hydrogen is performed with the hydrogen being retained in the molecule of MCH, which is a component of gasoline and diesel oil and is in the liquid state under normal temperature and normal pressure, and therefore, this method is highly safe in principle. Specifically, even if the tank and the reactor of the present system are caught in a fire, it would be similar to conventional oil refinery fires, and it is considered that the possibility of causing severe damage to the surrounding urban area is very low. The thought "accidents will eventually happen" is very important to safety measures, and that is why it is required to be safe in principle.

With this method, it is possible to store about 530 L of hydrogen gas in 1 L of liquid MCH. To physically decrease the volume of hydrogen gas to 1/500 or less, it is necessary to compress the hydrogen gas to 500 atm or higher or to cool the hydrogen gas to -253 °C or lower to make it liquid hydrogen which is 1/800 in volume, but according to the present method, by use of chemical reaction it is possible to decrease the volume to 1/500 under normal temperature and normal pressure. Also, since TOL and MCH are in the liquid state in a wide temperature range of -95 to 101 °C, they can be handled as a liquid such as water under any environment on the Earth. To establish a large-scale supply chain, it is necessary to procure hundreds of thousands of tons of TOL, but TOL is a fuel base material contained in high octane gasoline in a proportion of 10 % by weight or more and also is a general purpose chemical product produced in 20 million tons per year worldwide to be used widely as industrial solvent. Therefore, TOL can be easily procured in large amounts.

From the foregoing, the primary feature of the present method is high safety that can lower the potential risk regarding large-scale storage and transportation of hydrogen to the level of the risk related to conventional gasoline storage and transportation in principle, and this is the first reason why the applicant focused on this method. Also, storage of TOL and MCH in large tanks and transportation of the same by chemical tankers and chemical lorries have been practically implemented from old times as chemical products. In the current trend of electrification of automobiles, demand for gasoline and diesel oil as automobile fuels is expected to decrease and the existing infrastructure therefor, such as storage tanks, could be diverted. This can be a significant merit.

Further, in a case where hydrogen is used in large scale as fuel for power generation in the future, it is expected that hydrogen fuel reserve will become necessary as the current oil reserve. TOL and MCH do not chemically change even if stored for a long period and in large scale, and there is no extra energy consumption or loss due to long-term storage, and therefore, by storing MCH in the tanks of current oil reserve base, it is possible to convert the oil reserve bae to a reserve base for hydrogen energy.

The applicant focused on the organic chemical hydride method which has the highest safety and is advantageous in cost because the existing infrastructure can be diverted, started the development of novel dehydrogenation catalyst, which is the key to practical implementation, in 2002, and succeeded first in the world in developing novel dehydrogenation catalyst that can be industrially applicable to the organic chemical hydride method. Thereafter, for the purpose of establishment of technology for the whole system, the applicant used the developed catalyst in the dehydrogenation process and combined it with the toluene hydrogenation process, which realizes hydrogen storage reaction, to construct a demonstration plant which continuously repeats the hydrogen storage and hydrogen generation at the same place in 2013. The demonstration operation was conducted from April 2013 to November 2014 for about 10, 000 hours in total, and it was confirmed that high performance as designed could be maintained stably, whereby the establishment of the technology was completed.

Thereafter, as the final stage of the development, a first-in-the-world demonstration of international hydrogen supply chain, in which about 200 tons of hydrogen was actually transported from Brunei in Southeast Asia to Kawasaki waterfront in Japan by use of the present system, was carried out as a project of NEDO (New Energy and Industrial Technology Development Organization) in 2020, and the demonstration of transporting 100 tons or more of hydrogen per year using the present system has been completed.

Japan has adopted a policy to promote practical implementation and dissemination of hydrogen energy as a national policy from the Fourth Strategic Energy Plan after the Great East Japan Earthquake, and following formulation of the Hydrogen Fuel Cell Technology Roadmap, the Hydrogen Basic Strategy was approved by the cabinet in 2017. Practical implementation of the aforementioned organic chemical hydride method is incorporated, as a hydrogen energy carrier for "storing" and "transporting" hydrogen energy in a large scale, in the Hydrogen Basic Strategy, which sets 30 $¥/Nm^3$ as a target hydrogen supply price by 2030, and 20 $¥/Nm^3$ by 2050. This requires cost reduction by continuous improvement technology development, and improvement of the catalyst performance is an important element of the cost reduction. Thus, the present invention is effective in the practical implementation of the organic chemical hydride method and highly useful industrially.

Also, the egg shell-type platinum-loaded alumina catalyst and the uniform-type platinum-loaded alumina catalyst prepared by making sulfur contained in the $\gamma$-alumina carrier not only can be used as a catalyst but also can be effectively used as an adsorbent or the like. The catalysts of the present invention are useful in application to the organic chemical hydride method, and are also useful as a filler of guard columns for preprocessing of adsorbing impurities and the like by catalytic reaction process.

In this way, the egg shell-type platinum-loaded alumina catalyst and the uniform-type platinum-loaded alumina catalyst prepared by making the $\gamma$-alumina carrier contain sulfur according to the present invention can be favorably used in the dehydrogenation reaction of hydrogenated aromatics such as methylcyclohexane used as a hydrogen energy carrier, and can contribute to practical implementation of the hydrogen storage and transportation system according to the organic chemical hydride method. Besides, there is a possibility that they can be widely applied to the existing catalytic reaction processes in which the platinum-loaded alumina catalyst is used, and the industrial applicability is very high.

EFFECT OF THE INVENTION

According to the foregoing configuration, the platinum-loaded alumina catalyst according to the present invention has higher catalyst performance, particularly with respect to the catalyst life, compared to the conventional platinum-loaded alumina catalyst. Also, with the method of producing the platinum-loaded alumina catalyst according to the present invention, it is possible to mass-produce the catalyst easily by existing catalyst production facilities. Further, the platinum-loaded alumina catalyst according to the present invention not only can be used as an alternative to the existing platinum-loaded alumina catalyst, but also can be favorably used as a dehydrogenation catalyst for methylcyclohexane or the like in the organic chemical hydride method, which is one of the hydrogen storage and transportation technologies.

BRIEF DESCRIPTION OF THE DRAWINGS

Figure 1 is an explanatory diagram showing (A) a transmission electron micrograph in 2000s and (B) a transmission electron micrograph in recent years of a catalyst;
Figure 2 is an explanatory diagram of (A) an egg shell-type metal-loaded catalyst and (B) a uniform-type metal-loaded catalyst; and
Figure 3 is an explanatory diagram related to measurement of platinum particle diameters based on the transmission electron micrograph.

MODE(S) FOR CARRYING OUT THE INVENTION

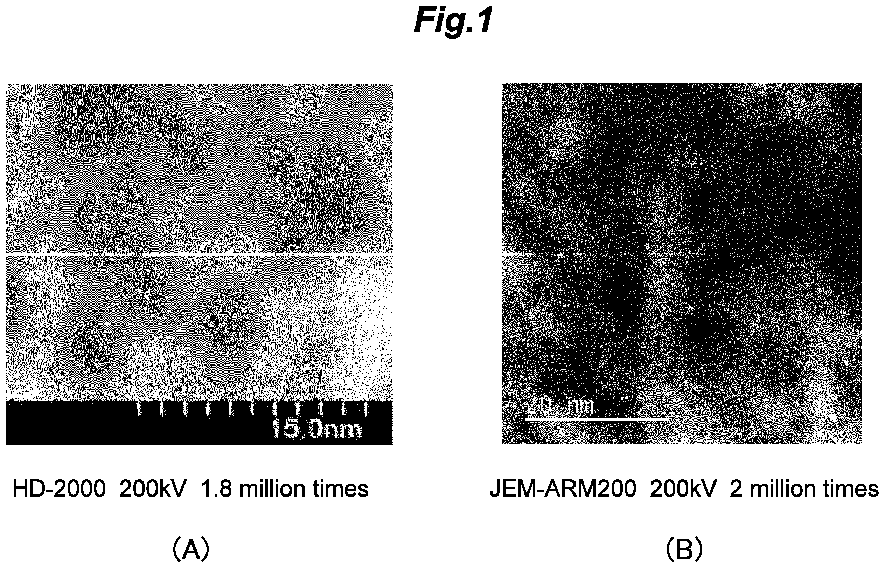

Figure 1 shows photographs of catalyst taken by (A) a transmission electron microscope in 2000s and (B) a transmission electron microscope in recent years. Figure 1(A) is a photograph of an egg shell-type catalyst disclosed in Patent Document 1 (JP4652695B2). The photograph of Figure 1(A) was taken at 1.8 million times magnification by using HITACHI, HD-200 electron microscope, which was a state-of-the-art transmission electron microscope at the time of 2006. Figure 1(B) is a photograph of a uniform-type platinum-loaded alumina catalyst disclosed in Patent Document 2 (JP4142733B2). The photograph of Figure 1(B) was taken at 2 million times magnification by using JEOL, JEM-ARM200 electron microscope in 2018.

With the resolution of a general transmission electron microscope in 2000s, it was not possible to measure platinum particle diameters of several nanometers by direct observation, and therefore, it was common at that time to estimate the particle diameters by CO-pulse method (CO pulse adsorption method). Currently, owing to the progress of electron microscope performance, it is possible to directly observe particles of several tenths of nanometer (Å) and molecules such as benzene rings with a resolution of about 0.1 nm (1 Å).

In the transmission electron micrograph in 2000s shown in Figure 1(A), it is seen that multiple platinum particles are each independently loaded but the outline of white dots (namely, platinum particles) is unclear and thus there is a tendency that the particles appear bigger than they actually are.

In the transmission electron micrograph in recent years shown in Figure 1(B), it is seen that the outline of white dots (the platinum particles) can be observed more clearly. Therefore, the particle diameters of the platinum loaded on the alumina carrier can be measured with higher accuracy by direct observation with the transmission electron microscope in recent years. The average particle diameter of the platinum particles in the platinum-loaded alumina catalyst according to the present invention can be obtained by measuring the particle diameters of a predetermined number (typically, about 50) of platinum particles in a photograph taken by the transmission electron microscope as shown in Figure 1(B) and calculating the average value thereof. In the transmission electron microscope, it is preferred to set the magnification such that the particle diameters of the predetermined number of platinum particles are fit in the photograph (for example, with the platinum-loaded alumina catalyst of the present invention, 40 to 50 platinum particles can be observed in the field of view at 2 million times magnification). The size of each platinum particle can be measured by aligning a measurement line with the outline of the particle on the computer screen equipped in the electron microscope system. At this time, in a case where the shape of the platinum particle has a long-axis diameter and a short-axis diameter, the long-axis diameter can be measured by aligning the measurement line with the outline of the long-axis diameter, and the short-axis diameter can be measured in a similar manner. Since there is substantially no difference between the long-axis diameter and the short-axis diameter for the platinum particles on the platinum-loaded alumina catalyst of the present invention, it is possible to use the long-axis diameter as a representative value. Also, it is also possible to measure the size of the particle diameters by printing the image and measuring the particle diameters with a ruler to compare with a scale on the image.

In contrast to this, the platinum particle diameters disclosed in the above-described Patent Document 1 and Patent Document 2 are estimated values according to the CO-pulse method. It can be considered that there is an error between the particle diameters estimated by the CO-pulse method and the particle diameters measured by direct observation using the transmission electron microscope. This is because in the CO-pulse method, the particle diameters are likely to be estimated smaller compared to the particle diameters measured by direct observation. In the CO-pulse method, since 1 molecule of CO is adsorbed on 1 atom of platinum on the platinum particle surface, a total CO adsorption amount is measured, and assuming that the shape of the platinum particle is a cube, the particle diameter is estimated as a length of one side thereof. At this time, the estimation is made with an assumption that CO is not adsorbed on the carrier. In the case of the platinum-loaded alumina catalyst, CO is preferentially adsorbed on platinum, and the injection of CO is stopped immediately when the amount of discharged CO becomes equal to the amount of injected CO, but in the alumina carrier, the surface area is large and a certain amount of CO is adsorbed on the carrier, and therefore, this CO is estimated to be adsorbed on the platinum surface.

Here, the CO-pulse method will be described. When CO is pulsatively injected into a sample, CO is adsorbed on the surface of loaded metal and the amount of discharged CO is small in the early stage of the injection. After a while, CO is adsorbed on almost the entire surface of the loaded metal, and when a steady state is reached, almost all of the injected CO is discharged. At this time, the amount of discharged CO during adsorption is subtracted from the amount of discharged CO in the steady state, and the sum of the differences is obtained as the CO adsorption amount. The CO-pulse method is a method for calculating a metal surface area, dispersion ratio, and particle diameter from the adsorption amount and the loaded metal content. A concrete calculation method is described below.

From the CO gas amount Vt adsorbed by a sample amount of catalyst W (g) at a measurement temperature, the adsorption gas amount V per g of the catalyst at 0 °C was obtained from the following equation (1).

$$V = (Vt/W) \times \{273/(273 + t)\} \text{ (ml/g-cat) ... (1)}$$

Here, when the percentage of metal content of the sample is defined as C (%) and the atomic weight of the loaded metal is defined as M, the number of moles R of the loaded metal per g of the sample is obtained from the equation (2).

$$R = (C/100) \times (1/M) \text{ (mol/g-cat) ... (2)}$$

the number of moles K of the adsorption gas amount per g of the sample is obtained from the equation (3).

$$K = V/ (22.4 \times 10^{-3} \times 10^{6}) \text{ (mol/g-cat) ...(3)}$$

From these, the dispersion degree B (proportion of effective surface metal in the loaded metal) is obtained from the equation (4).

$$B = (K/R) \times 100 \text{ (\%) ... (4)}$$

When the lattice constant of the loaded metal catalyst is defined as a (0.1 nm or 1 Å), and it is assumed that one adsorption gas molecule is adsorbed to a lattice constant area $a^2$, the specific surface area S of the metal is obtained from the equation (5).

$$S = \text{the number of gas molecules adsorbed to 1 g of sample} \times a^2$$

$$= K \times 6.02 \times 10^{23} \times (a \times 10^{-10})^2 \text{ ... (5)}$$

Further, when a loaded metal particle is assumed to be a cube with a side length D (m), five surfaces out of six surfaces of the particle are effective, and therefore, the following equations are established.

$$\text{Effective area S of one particle} = 5D^2 \text{ (m}^2\text{) ... (6)}$$

$$\text{Volume v of one particle} = D^3 \text{ (m}^3\text{) ... (7)}$$

When the number of particles of the loaded metal per g of sample is defined as n, the following equations are established.

$$\text{Specific surface area S of loaded metal} = ns = n5D^2 \ (m^2) \ ... \ (8)$$

$$\text{Volume Vc of loaded metal} = nv = Nd^3 \ (m^3) \ ... \ (9)$$

From the equations (6) to (9), the length D (m) of one side is expressed by the equation (10).

$$S/Vc = 5/D, \text{ and therefore, } D = 5Vc/S \ (m) \ ... \ (10)$$

Here, when the percentage of loaded metal content is defined as C (%) and the specific gravity is defined as d $(g/cm^3)$, the volume Vc of the loaded metal per g of sample is expressed by the equation (11).

$$Vc = \text{loaded metal weight per g of sample (g/g) / specific gravity of loaded metal}$$

$$(g/cm^3)$$

$$= C/100/d \ (g/cm^3) \ ... \ (11)$$

Accordingly, the particle diameter is calculated from the equation (12).

$$\text{particle diameter} = 5Vc/S$$

$$= \{5 \ (C/100/d) \times 10^{-6}\}/S \ (m)$$

$$= \{5 \ (C/100/d) \times 10^{-6} \times 10^{10}\}/S \ (\text{Å}) \ ... \ (12)$$

Note: 1 Å = 0.1 nm.

As described above, in the conventional platinum-loaded alumina catalyst, the particle diameter of the platinum loaded on the alumina carrier was measured (calculated) using the CO-pulse method or the transmission electron micrograph at that time (see Figure 1(A)), in which error was relatively large. Therefore, in the conventional platinum-loaded alumina catalyst, the preferred range of the platinum particle diameter was set as a relatively wide range because it was difficult to control the platinum particle diameter with high accuracy.

In contrast to this, in the present invention, based on the value of the platinum particle diameter measured with relatively high accuracy according to the transmission electron micrograph in recent years (see Figure 1(B)), a range of the platinum particle diameter (platinum particle diameter distribution) in which the catalyst life can be remarkably improved compared to the conventional platinum-loaded alumina catalyst is set. In the platinum-loaded alumina catalyst according to the present invention, it is preferred that 70 % or more of the particles of platinum loaded on the $\gamma$-alumina carrier have a size of 0.8 to 1.5 nm (8 to 15 Å) in direct observation using the transmission electron microscope. More preferably, 80 % or more of the particles of platinum loaded on the $\gamma$-alumina carrier have a size of 0.8 to 1.5 nm (8 to 15 Å). Further preferably, 90 % or more of the particles of platinum loaded on the $\gamma$-alumina carrier have a size of 0.8 to 1.5 nm (8 to 15 Å).

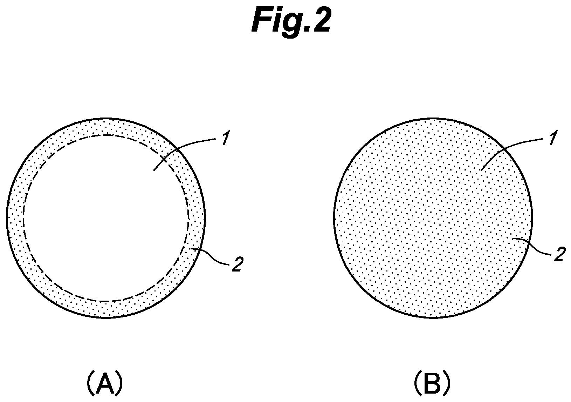

Next, an egg shell-type metal-loaded catalyst and a uniform-type metal-loaded catalyst of the present invention will be described with reference to Figure 2. The egg shell-type metal-loaded catalyst refers to a state where a metal member to be loaded is dispersed and loaded only on the outer shell part of the cross section of a molded catalyst. Namely, a metal loading part 2 on which the metal member is loaded is formed in the outer shell part of a porous carrier 1. The uniform-type metal-loaded catalyst refers to a state where a metal member is dispersed over the entire cross section of the catalyst and the metal loading part 2 on which the metal member is loaded is formed over the entire inside of a molded body of the porous carrier 1.

The platinum-loaded alumina catalyst according to the present invention includes an alumina carrier and platinum loaded on the alumina carrier.

Next, the alumina carrier used in the platinum-loaded alumina catalyst according to the present invention will be described.

The alumina carrier is a porous $\gamma$-alumina carrier. More specifically, as disclosed in JPH6-72005B2, for example, the alumina carrier preferably is a porous $\gamma$-alumina carrier obtained by washing by filtration a slurry of aluminum hydroxide generated by neutralizing aluminum salt, dehydrating and drying the obtained alumina hydrogel, and then calcining the resultant at 400 to 800 °C for about 1 to 6 hours. More preferably, the alumina carrier is a porous γ-alumina carrier obtained through a pH swing process in which the pH of alumina hydrogel is alternately fluctuated between a pH range of the dissolution of alumina hydrogel and a pH range of the precipitation of boehmite gel and simultaneously an alumina hydrogel forming substance is added for growing crystals of the alumina hydrogel when the pH is fluctuated from at least either one of the pH ranges to the other one of the pH ranges. The porous γ-alumina carrier obtained through the pH swing process is excellent in the uniformity of pore distribution, and excellent in that the physical properties of each pellet are stable because there is less variation in the physical properties also in the alumina carrier pellet after the formation of the carrier.

The inventors of the present application made further study on the relationship between the drying and calcining conditions of the alumina hydrogel (boehmite) and the particle diameters of the loaded platinum and, as a result, found that to stably load many of the platinum particles on the γ-alumina carrier to have a size in the range of 0.8 to 1.5 nm (8 to 15 Å), it is particularly preferred that the drying temperature is 200 °C or lower, the temperature of the calcination performed thereafter is 250 to 400 °C, and the calcination time is 1 to 12 hours.

When preparing the uniform-type platinum-loaded alumina catalyst according to the present invention, there is no limitation on the sulfur or sulfur compound to be dispersed in the alumina carrier beforehand for incorporation thereof in so far as the sulfur or sulfur compound has a sulfur element and can be uniformly dispersed in the catalyst carrier during the preparation of the catalyst carrier of after the preparation of the catalyst carrier. For example, sulfur crystal powders, and sulfur-containing compounds such as sulfuric acid, and sulfate including ammonium sulfate can be mentioned as the sulfur or sulfur compound. From the viewpoint that sulfur is likely to disperse on a carrier, sulfur compounds having solubility in water or an organic solvent are preferable, and sulfuric acid, ammonium sulfate, etc. can be mentioned as such sulfur compounds.

The amount of sulfur to be contained in a carrier is preferably 0.15 to 5.0 % by weight (wt%), and more preferably 0.15 to 3.0 % by weight, calculated as elemental sulfur (S). When the sulfur content is less than 0.15 % by weight, the degree that metal is uniformly loaded as far as the center of the catalyst is low, while when the sulfur content exceeds 5 % by weight, a problem is likely to occur that sulfur is likely to locally agglomerate and metal is not dispersed and loaded on such a portion. In view of the above, the most suitable sulfur content range is 0.15 to 5.0 % by weight considering the effect that metal is uniformly dispersed and loaded.

The inventors of the present application made further study on the relationship between the sulfur concentration and the particle diameters of the loaded platinum and, as a result, found that to stably load many of the platinum particles on the γ-alumina carrier to have a size in the range of 0.8 to 1.5 nm (8 to 15 Å), it is particularly preferred that with respect to the range of sulfur content, sulfur or a sulfur compound is contained in a range of 0.5 to 1.2 % by weight calculated as elemental sulfur (S).

In the present invention, with respect to a method of preparing a sulfur-containing catalyst carrier containing the above-mentioned sulfur or sulfur compound, usable is a method capable of incorporating the sulfur or sulfur compound in a state where the sulfur or sulfur compound is uniformly dispersed throughout the cross section of the carrier. For example, the following methods are mentioned: method A involving kneading sulfur powder in a metal hydroxide gel serving as a precursor of a metal oxide obtained when preparing a catalyst carrier, forming the resultant into a predetermined shape, and drying and calcining the resultant; method B involving preparing a metal hydroxide gel serving as a precursor of a metal oxide containing sulfur using metal sulfate and/or sulfuric acid when preparing a catalyst carrier, forming the resultant into a predetermined shape, and drying and calcining the resultant; method C involving forming a metal hydroxide gel serving as a precursor of a metal oxide into a predetermined shape when preparing a catalyst carrier, drying the resultant to form a dry metal hydroxide gel, impregnating the dry metal oxide with a sulfur compound solution, and calcining the same; method D involving forming a metal hydroxide gel serving as a precursor of a metal oxide into a predetermined shape when preparing a catalyst carrier, drying the resultant to form a dry metal hydroxide, impregnating the dry metal hydroxide with a sulfur compound solution, and calcining the same; and method E involving forming a metal hydroxide gel serving as a precursor of a metal oxide into a predetermined shape, drying the resultant to form a dry metal hydroxide gel, calcining the dry metal hydroxide gel to form a calcined metal oxide, impregnating the calcined metal oxide with a sulfur compound solution such as a sulfuric acid aqueous solution and an ammonium sulfate solution, and further calcining the resultant.

The inventors of the present application made further study on the method for preparing a sulfur-containing catalyst carrier and, as a result, found that to stably load many of the platinum particles on the γ-alumina carrier to have a size in the range of 0.8 to 1.5 nm (8 to 15 Å), it is particularly preferred to disperse and load sulfur on the surface of the γ-alumina carrier according to the aforementioned method E.

With respect to calcining conditions when preparing the sulfur-containing catalyst carrier, usually, the calcining temperature is 100 to 1000 °C, and preferably 350 to 800 °C, and the calcining time is 0.5 to 48 hours, and preferably 1 to 24 hours. When the calcining temperature is lower than 350 °C, conversion to an oxide from a hydroxide may not be fully performed, while when the calcining temperature is higher than 800 °C, the surface area after calcining may be dramatically reduced.

The inventors of the present application made further study on the drying and calcining conditions when preparing a sulfur-containing $\gamma$-alumina carrier and, as a result, found that to stably load many of the platinum particles on the $\gamma$-alumina carrier to have a size in the range of 0.8 to 1.5 nm (8 to 15 Å), it is particularly preferred that with respect to the drying condition, the drying temperature is 100 to 200 °C and the drying time is 3 to 12 hours, and with respect to the calcination condition, the calcination temperature 250 to 400 °C and the calcination time is 1 to 12 hours.

In the present invention, the amount of platinum to be loaded on the aforementioned sulfur-containing catalyst carrier is 0.05 to 5.0 % by weight, preferably 0.1 to 3.0 % by weight, calculated as elemental platinum. When the loading amount of platinum is less than 0.05 % by weight, there is a problem that the activity is low, while when the loading amount of platinum exceeds 5.0 % by weight, there are problems that the particle diameter of platinum increases, the selectivity is reduced, sintering is likely to occur, resulting in that deactivation is likely to occur.

The inventors of the present application made further study on the preferred loading amount of platinum and, as a result, found that to stably load many of the platinum particles on the $\gamma$-alumina carrier to have a size in the range of 0.8 to 1.5 nm (8 to 15 Å), the loading amount of platinum is preferably 0.1 to 1.5 % by weight calculated as content of elemental platinum, and more preferably, 0.5 to 1.5 % by weight from the viewpoint of improvement of life of the prepared platinum-loaded alumina catalyst.

In the present invention, when platinum metal is loaded on the $\gamma$-alumina carrier, the above-mentioned $\gamma$-alumina carrier may be impregnated with a solution of platinum compound, dried, and then calcined at a predetermined temperature. As the platinum compound, chloride, bromide, ammonium salt, carbonyl compound, or various complex compounds, such as an amine complex, an ammine complex, and an acetylacetonato complex, of platinum can be mentioned. The platinum compound may be, for example, chloroplatinic acid, platinum acetylacetonate, ammonium platinate, bromo platinate, platinum dichloride, platinum tetrachloride hydrate, platinum carbonyl dichloride, dinitrodiamine platinate, or the like.

The inventors of the present application made further study on the platinum compound for impregnation and, as a result, found that from the viewpoint of improvement of life of the prepared platinum-loaded alumina catalyst, it is particularly preferred that the $\gamma$-alumina carrier after calcination is impregnated with platinum by using a chloroplatinic acid aqueous solution as a platinum reagent aqueous solution.

After the alumina carrier is impregnated with the above-mentioned solution of platinum compound, the alumina carrier to which the platinum compound adheres is dried at 50 to 200 °C for 0.5 to 48 hours, and thereafter is calcined at 350 to 600 °C for 0.5 to 48 hours, more preferably at 350 to 450 °C for 0.5 to 5 hours.

The inventors of the present application made further study on the drying and calcining conditions after suitable platinum impregnation to the alumina carrier (for example, the content calculated as elemental platinum is in a range of 0.5 to 1.5 % by weight) and, as a result, found that to stably load many of the platinum particles on the $\gamma$-alumina carrier to have a size in the range of 0.8 to 1.5 nm (8 to 15 Å), it is particularly preferred that with respect to the drying condition, the drying temperature is 100 to 200 °C and the drying time is 3 to 12 hours, and with respect to the calcination condition, the calcination temperature is 250 to 450 °C and the calcination time is 1 to 8 hours.

Then, as a final step of the platinum loading process, the alumina carrier to which the platinum compound adheres is placed in a hydrogen gas atmosphere and a hydrogen reduction process is performed under reduction condition at 350 to 600 °C for 0.5 to 48 hours, preferably at 350 to 550 °C for 3 to 24 hours. If the temperature during hydrogen reduction is lower than 350 °C, a problem that platinum is not sufficiently reduced occurs, and if the temperature exceeds 600 °C, a problem that the platinum particles are sintered during reduction and the metal dispersion degree is lowered occurs.

The inventors of the present application made further study on the temperature condition of the hydrogen reduction after suitable platinum impregnation and calcination and, as a result, found that to stably load many of the platinum particles on the $\gamma$-alumina carrier to have a size in the range of 0.8 to 1.5 nm (8 to 15 Å), it is particularly preferred that the temperature of the hydrogen reduction is 300 to 450 °C and is lower than or equal to the temperature for calcination after platinum impregnation and that the hydrogen reduction time is 1 to 15 hours.

The amount of alkali that is added to the aforementioned egg shell-type platinum-loaded alumina catalyst and the uniform-type platinum-loaded alumina catalyst prepared by making sulfur contained in the $\gamma$-alumina carrier is 0.1 to 5 % by weight, preferably 0.3 to 3.0 % by weight, and more preferably 0.5 to 1.5 % by weight. When the loading amount of alkali metal is less than 0.1 % by weight, there is a problem that the catalyst life is short and the effect is low, while when the loading amount is more than 5.0 % by weight, there is a problem that the activity is lowered and the catalyst life is shortened.

The inventors of the present application made further study on a preferred addition amount of alkali in loading many of the platinum particles on the $\gamma$-alumina carrier to have a size in the range of 0.8 to 1.5 nm (8 to 15 Å) and, as a result, found that so long as the addition amount of alkali is 0.5 to 1.5 % by weight, there is no significant influence on the size of the platinum particles after preparation.

The compound of alkaline metal used when loading the alkaline metal to the egg shell-type platinum-loaded alumina catalyst and the uniform-type platinum-loaded alumina catalyst prepared by making sulfur contained in the $\gamma$-alumina carrier may be, for example, a chloride, bromide, iodide, nitrate, sulfate, acetate, propionic acid, and the like of, the alkaline metal, which preferably is water-soluble and/or soluble to an organic solvent such as acetone. Such a compound may be, for example, sodium chloride, sodium bromide, sodium iodide, sodium nitrate, sodium sulfate, sodium acetate, sodium propionate, potassium chloride, potassium bromide, potassium iodide, potassium nitrate, potassium sulfate, potassium acetate, potassium propionate, calcium chloride, calcium bromide, calcium iodide, calcium nitrate, calcium sulfate, calcium acetate, calcium propionate, or the like.

Also, when the alkaline metal is loaded on the egg shell-type platinum-loaded alumina catalyst and the uniform-type platinum-loaded alumina catalyst prepared by making sulfur contained in the $\gamma$-alumina carrier, they are impregnated with a solution of a compound of the alkaline metal, thereafter dried under a drying condition at room temperature to 200 °C for 0.5 to 48 hours, preferably at 50 to 150 °C for 0.5 to 24 hours, more preferably at 80 to 120 °C for 0.5 to 5 hours, and then calcined at 350 to 600 °C for 0.5 to 48 hours, preferably 350 to 450 °C for 0.5 to 5 hours.

The inventors of the present application made further study on the drying condition after the impregnation with a solution of a preferred alkali compound in loading many of the platinum particles on the $\gamma$-alumina carrier to have a size in the range of 0.8 to 1.5 nm (8 to 15 Å) and, as a result, found that so long as the temperature is at room temperature to 200 °C, there is no influence on the size of the loaded platinum particles irrespective of the drying time.

The dried matter on which the alkali metal is loaded, which is obtained by impregnating the alkali metal into the egg shell-type platinum-loaded alumina catalyst and the uniform-type platinum-loaded alumina catalyst prepared by making sulfur contained in the $\gamma$-alumina carrier and drying the impregnated catalyst, is not calcined thereafter, and is directly subjected to final hydrogen reduction. The reduction condition of this hydrogen reduction is preferably at 350 to 600 °C for 0.5 to 48 hours, more preferably at 350 to 550 °C for 3 to 24 hours in a hydrogen gas atmosphere. If calcination is performed prior to the hydrogen reduction of the dried matter on which the alkali metal is loaded, there arises a problem that the catalyst performance related to activity, selectivity, and life is lowered. Also, if the temperature at the time of the hydrogen reduction is lower than 350 °C, there arises a problem that platinum is not fully reduced, and if the temperature at the time of the hydrogen reduction exceeds 600 °C, there arises a problem that sintering of platinum particles occurs at the time of reduction, and the metal dispersion degree is lowered.

The inventors of the present application made further study on the hydrogen reduction condition after the impregnation with a solution of a preferred alkali compound and drying in loading many of the platinum particles on the $\gamma$-alumina carrier to have a size in the range of 0.8 to 1.5 nm (8 to 15 Å) and, as a result, found that if the temperature and the reduction time are less than or equal to the temperature and the reduction time of the hydrogen reduction carried out as the final step of the platinum loading process before addition of the alkali metal, there is no influence on the size of the loaded platinum particles.

Hereinafter, preferable embodiments of the present invention will be specifically described based on Examples and Comparative Examples.

[Comparative Example 1] (comparison of measurement results of particle diameters of the egg shell-type catalysts described in Patent Document 1 and the uniform-type platinum-loaded alumina catalysts described in Patent Document 2 between direct observation using a transmission electron microscope and the CO-pulse method)

The platinum particle diameters of the egg shell-type catalysts described in Patent Document 1 are particle diameters estimated based on the dispersion degree estimated from the CO adsorption amount measured by the CO-pulse method with the assumption that the shape of the platinum particle is a cube and, as shown in Table 2 (Experimental Example 1) and Table 3 (Experimental Example 2) of Patent Document 1, were estimated as particle diameters in a range of 0.55 to 1.4 nm (5.5 to 14 Å).

On the other hand, the particle diameters of the uniform-type platinum-loaded alumina catalysts described in Patent Document 2 were estimated as particle diameters of 0.65 to 1.1 nm (6.5 to 11 Å), as shown in Table 1 (Embodiment 4) of Patent Document 2.

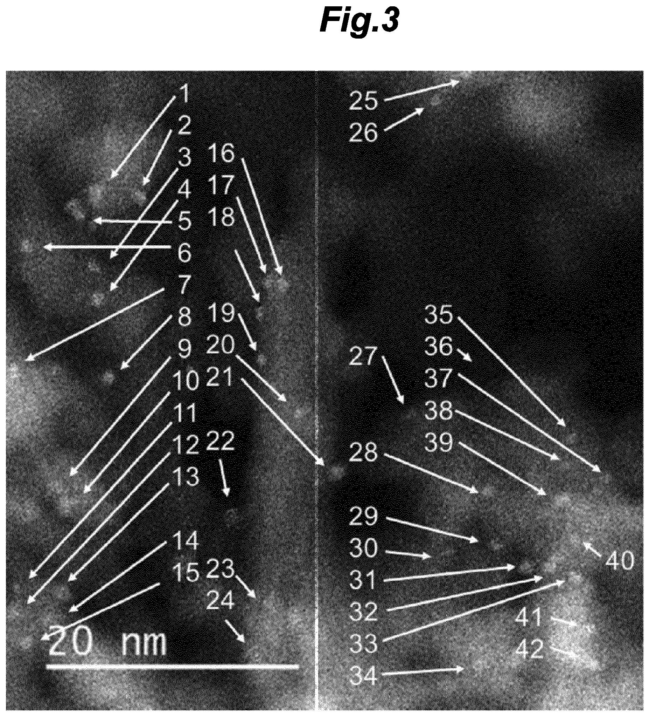

As shown in Figure 3, by direct observation using a transmission electron microscope, the particle diameters of 42 platinum particles (see the numbers 1-42 in Figure 3) in a uniform-type platinum-loaded alumina catalyst described in Patent Document 2 were measured. The image of the catalyst shown in Figure 3 corresponds to the transmission electron micrograph shown in Figure 1(B). Also, this catalyst corresponds to the catalyst No.6 (the particle diameter is 0.65 nm (6.5 Å)) described in Patent Document 2 (Table 1).

The measurement of platinum particle diameters can be performed by using a particle diameter measurement function on the screen of the electron microscope. Note, however, that it is also possible to obtain substantially the same measurement result by comparing the length of a part of the particle diameter having the largest diameter with a scale shown in the electron micrograph. Table 1 shows the measurement result of the particle diameters. The average particle diameter of the 42 platinum particles shown in Table 1 was 1.68 nm (16.8 Å).

[Table 1]

| No. | particle diameter (nm) | No. | particle diameter (nm) | No. | particle diameter (nm) | No. | particle diameter (nm) | No. | particle diameter (nm) |
|---|---|---|---|---|---|---|---|---|---|
| 1 | 2.0 | 11 | 0.8 | 21 | 1.6 | 31 | 1.7 | 41 | 1.2 |
| 2 | 1.8 | 12 | 1.6 | 22 | 2.2 | 32 | 1.7 | 42 | 1.3 |
| 3 | 1.5 | 13 | 1.4 | 23 | 1.4 | 33 | 1.8 | 43 | - |
| 4 | 2.0 | 14 | 1.5 | 24 | 1.3 | 34 | 1.2 | 44 | - |
| 5 | 2.3 | 15 | 1.4 | 25 | 1.5 | 35 | 1.3 | 45 | - |
| 6 | 1.3 | 16 | 1.5 | 26 | 1.4 | 36 | 1.2 | 46 | - |
| 7 | 1.4 | 17 | 1.1 | 27 | 1.3 | 37 | 1.1 | 47 | - |
| 8 | 1.2 | 18 | 1.3 | 28 | 1.2 | 38 | 1 | 48 | - |
| 9 | 1.7 | 19 | 1.2 | 29 | 1.2 | 39 | 1.9 | 49 | - |
| 10 | 2.0 | 20 | 1.0 | 30 | 1.3 | 40 | 1.3 | 50 | - |

According to the measurement result of the platinum particle diameters shown in Table 1, it is seen that, of the 42 platinum particles measured, 19 (about 45 %) platinum particles had a size in a range of 0.8 to 1.5 nm (8 to 15 Å) , and 23 platinum particles were larger than 1.5 nm (15 Å) and had a size of 1.6 nm (16 Å) or larger.

Thus, when measured from the image for direct observation of the uniform-type platinum-loaded alumina catalyst described in Patent Document 2 (the catalyst No.6 in Table 1 of Patent Document 2) taken by the electron microscope, the average particle diameter of platinum was 1.68 nm (16.8 Å). From this, it is seen that the estimated value 0.65 nm (6.5 Å) of the particle diameter of platinum according to the CO-pulse method described in Patent Document 2 is a remarkably small value compared to the value measured by direct observation of the electron micrograph.

As described above, with respect to the egg shell-type catalysts described in Patent Document 1 and the uniform-type platinum-loaded alumina catalysts described in Patent Document 2, the particle diameters estimated based on the dispersion degree estimated from the CO adsorption amount measured by the CO-pulse method and the assumption that the shape of the platinum particle is a cube were estimated as particle diameters of 0.55 to 1.4 nm (5.5 to 14 Å), but these particle diameters also are considered to be fairly small values according to the direct observation using the electron microscope.

The large error of the estimated value of the particle diameter obtained by the CO-pulse method like this is considered to be attributed to that in the CO-pulse method, the estimation is made on an assumption that the introduced CO is adsorbed on the platinum atoms exposed on the surface of platinum particles but actually there are many CO molecules adsorbed on the alumina carrier and therefore the CO adsorption amount is observed to be larger, and that the shape of the platinum particle is assumed to be a cube and the particle diameter is estimated as a length of one side thereof.

[Comparative Example 2] (preparation method described in an embodiment of Patent Document 1)

A preparation method of an egg shell-type platinum-loaded $\gamma$-alumina catalyst described in an embodiment of Patent Document 1 is explained.

Similarly to the embodiment of Patent Document 1, a porous $\gamma$-alumina carrier was produced according to the conventional technology described in Embodiment 1 of JPH6-72005B2. An outline of this method is as follows. A sodium aluminate aqueous solution was instantaneously added in hot dilute sulfuric acid while being vigorously stirred to obtain an aluminum hydroxide slurry suspension (pH10). This suspension was used as seed aluminum hydroxide and while stirring was continued, an operation of alternately adding the hot dilute sulfuric acid and the sodium aluminate aqueous solution at a constant interval was repeated to obtain filtered and washed cake. This cake was extruded and dried, and thereafter was calcined at 500 °C for 3 hours.

The $\gamma$-alumina carrier thus prepared physical properties of a surface area of 240 $m^2$/g, a pore volume of 0.713 $cm^3$/g, an average pore diameter of 11.9 nm (119 Å), and an occupancy of pores with pore diameters 9.0 to 30.0 nm (90 to 300 Å) of 90 %. 79g of 0.4 wt%-chloroplatinic acid aqueous solution prepared so that the pH value was 2.0 was added to 20g of this porous $\gamma$-alumina carrier, and this was left for 3 hours for impregnation before water was removed by decantation. Subsequently, the resultant was dried for 3 hours at 120 °C and then was calcined for 3 hours at 400 °C in a muffle furnace under air flow. The obtained calcined matter was cooled to normal temperature in the desiccator and thereafter was reduced at 400 °C for 15 hours under hydrogen flow to prepare a dehydrogenation catalyst (corresponds to the catalyst No.2 in Table 2 of the embodiment of Patent Document 1). The estimated value of the platinum particle diameter of this catalyst according to the CO-pulse method was 0.55 nm (5.5 Å).

[Comparative Example 3] (preparation method described in an embodiment of Patent Document 2)

A preparation method of a uniform-type platinum-loaded γ-alumina catalyst described in an embodiment of Patent Document 2 is explained.

3900 cc of aluminum nitrate aqueous solution with a concentration of 2.67 mol/L was prepared and simultaneously, 3900 cc of 14 % aqueous ammonia solution was prepared. 20 L of pure water was put in a 30-L enamel container, and the container was warmed to 70 °C under stirring. While continuing stirring, a pH swing operation in which 1300 cc of aluminum nitrate aqueous solution was added, followed by stirring for 5 minutes (pH = 2.0), and thereafter, 1300 cc of aqueous ammonia solution was added, followed by stirring for 5 minutes (pH = 7.4) was performed 4 times. An aqueous slurry solution of the obtained aluminum hydroxide was filtered to recover a cake, and subsequently, a washing operation in which the cake was re-dispersed in 20 L of pure water and was filtered again was performed 3 times, obtaining a washed gel.

The washed cake was air dried to adjust the moisture, and then was formed into a rod-like shape having a diameter of 1.6 mm with an extruder. The resultant was dried (120 °C, 3 hours), crushed to about 1 cm in length, and calcined in a muffle furnace (500 °C, 3 hours), thereby yielding an alumina carrier A containing no sulfur. The obtained alumina carrier A had a BET surface area of 275 $m^2$/g and a pore volume of 0.65 $cm^3$/g as measured by mercury porosimetry. Also, the obtained alumina carrier A had an average pore diameter of 8.9 nm and had a sharp pore distribution in which almost all of the pores were concentrated near the average pore diameter. In addition, the volume occupied by pores having a diameter of 7 to 10 nm was 80 % or more of the total pore volume.

The alumina carrier A was impregnated with an ammonium sulfate aqueous solution with a concentration of 0.38 mol/L so that the sulfur content after calcination was 0.5 % by weight, and the solvent was removed with an evaporator. Thereafter, the alumina carrier A was dried (120 °C, 3 hours) and calcined (500 °C, 3 hours), thereby yielding an alumina carrier containing sulfur at 0.5 % by weight.

The alumina carrier thus prepared was impregnated with a chloroplatinic acid aqueous solution whose pH was adjusted to 2.0 so that the loading amount of platinum after calcination was 0.6 % by weight. Thereafter, moisture was removed with an evaporator, and the resultant was dried (120 °C, 3 hours) and calcined (400 °C, 3 hours). Then, the resultant was charged in a flow-type hydrogen-reducing apparatus, and hydrogen reduction was carried out at 450 °C for 15 hours in a hydrogen stream, thereby yielding a 0.6 wt% platinum-loaded alumina catalyst. The estimated value of the platinum particle diameter of this platinum-loaded alumina catalyst according to the CO-pulse method was 0.65 nm (6.5 Å).

[Comparative Example 4] (reaction test method related to the egg shell-type catalyst described in Patent Document 1 and the uniform-type catalyst described in Patent Document 2)

Comparison of dehydrogenation reaction test methods and reaction test results related to the egg shell-type catalyst described in Patent Document 1 and the uniform-type catalyst described in Patent Document 2 is shown. Here, the dehydrogenation reaction test described in Patent Document 1 and the dehydrogenation reaction test described in Patent Document 2 differ with respect to the condition of reaction temperature and concentration of hydrogen supplied with the raw material MCH. This is because the deterioration speed is different between the dehydrogenation catalyst described in Patent Document 1 and the dehydrogenation catalyst described in Patent Document 2. The reaction test condition of the deterioration speed at the time of development of the dehydrogenation catalyst described in Patent Document 1 was a condition in which deterioration was relatively hard to progress. Specifically, the reaction test condition described in Patent Document 1 was a condition with a reaction temperature of 300 °C and a hydrogen supply concentration of 20 %, whereas the reaction condition described in Patent Document 2 was a condition with a reaction temperature of 320 °C and a hydrogen supply concentration of 5 %. The reason for this is that since the dehydrogenation catalyst described in Patent Document 2 is a catalyst having a low deterioration speed and hard to deteriorate, the reaction test was performed under an accelerated condition in which deterioration is easy to progress.

In this Comparative Example, with respect to the dehydrogenation reaction test of methylcyclohexane (MCH), the results of dehydrogenation reaction tests using the dehydrogenation catalyst described in Patent Document 1 as the catalyst NO.1 and the dehydrogenation catalyst described in Patent Document 2 as the catalyst NO.2 are shown.

10 cc of each catalyst described above was put in a stainless steel reaction tube, which had an inside diameter of 12.6 mm and a length of 300 mm and which was equipped with a protection tube for a thermocouple whose outer dimension was 1/8 inch in the center of the cross section of the reaction tube, such that the center of the catalyst layer was positioned in a lengthwise center of the reaction tube, and 10 cc of spherical α-alumina beads with a diameter of 1 mm was placed on the upper side of the catalyst as a preheating layer. Under hydrogen flow (LHSV = 5.0; 50 cc/hr), temperature was raised until the central temperature of the catalyst layer reaches 320 °C. Subsequently, methylcyclohexane (MCH) in an amount corresponding to LHSV = 2.0 (20 cc/hr) was supplied to the reactor with a liquid supply pump for high-speed liquid chromatography (HPLC) (HPLC pump), and immediately, the flow rate of hydrogen was adjusted so that the hydrogen gas amount was 5 mol% with respect to the total amount of MCH and hydrogen gas. The reaction test was performed while adjusting the output of an electric furnace so that the central temperature of the catalyst layer was 320 °C during the reaction.

A gas-liquid separator was provided at the outlet of the reaction tube, and the resultant was separated into a liquid product such as toluene and gas such as hydrogen gas, which were generated by the dehydrogenation reaction, and the collected liquid product and gas were separately analyzed by gas chromatography.

The MCH conversion rate (%), toluene selectivity (%), toluene yield (%), and produced methane concentration (ppm) 24 hours after and 300 hours after the initiation of the reaction were obtained. The results are shown in Table 2.

[Table 2]

| catalyst No. | sulfur content (wt%) | plutinum loading amount (wt%) | after 24 hours from initiation of reaction | | | | after 300 hours from initiation of reaction | | | |
|---|---|---|---|---|---|---|---|---|---|---|
| | | | MCH conversion rate (%) | toluene selectivity (%) | toluene yield (%) | produced methane concentration (ppm) | MCH conversion rate (%) | toluene selectivity (%) | toluene yield (%) | produced methane concentration (ppm) |
| 1 | 0 | 0.6 | 98.2 | 99.88 | 98.1 | 180 | 94.5 | 99.9 | 94.4 | 115 |
| 2 | 0.5 | 0.6 | 98.2 | 99.92 | 98.1 | 50 | 97.7 | 99.93 | 97.6 | 35 |

[Example 1] (preparation method and particle diameter measurement result of the eff shell-type catalyst according to the present invention)

3900 cc of aluminum nitrate aqueous solution with a concentration of 2.67 mol/L was prepared and simultaneously, 3900 cc of 14 % aqueous ammonia solution was prepared. 20 L of pure water was put in a 30-L enamel container, and the container was warmed to 70 °C under stirring. While continuing stirring, a pH swing operation in which 1300 cc of aluminum nitrate aqueous solution was added, followed by stirring for 5 minutes (pH = 2.0), and thereafter, 1300 cc of aqueous ammonia solution was added, followed by stirring for 5 minutes (pH = 7.4) was performed 4 times. An aqueous slurry solution of the obtained aluminum hydroxide was filtered to recover a cake, and subsequently, a washing operation in which the cake was re-dispersed in 20 L of pure water and was filtered again was performed 3 times, obtaining a washed gel.

The washed cake was air dried to adjust the moisture, and then was formed into a rod-like shape having a diameter of 1.6 mm with an extruder. The resultant was dried (120 °C, 3 hours), crushed to about 1 cm in length, and calcined in a muffle furnace (350 °C, 3 hours), thereby yielding an alumina carrier A containing no sulfur. The obtained alumina carrier A had a BET surface area of 290 $m^2$/g and a pore volume of 0.61 $cm^3$/g as measured by mercury porosimetry. Also, the obtained alumina carrier A had an average pore diameter of 9.5 nm (95 Å) and had a sharp pore distribution in which almost all of the pores were concentrated near the average pore diameter. In addition, the volume occupied by pores having a diameter of 7 to 11 nm (70 to 110 Å) was 80 % or more of the total pore volume.

The alumina carrier thus prepared was impregnated with a chloroplatinic acid aqueous solution whose pH was adjusted to 2.0 so that the loading amount of platinum after calcination was 0.6 % by weight. Thereafter, moisture was removed with an evaporator, and the resultant was dried (120 °C, 3 hours) and calcined (350 °C, 3 hours). Then, the alumina carrier was placed in a flow-type hydrogen-reducing apparatus, and hydrogen reduction was carried out at 400 °C for 15 hours in a hydrogen stream, thereby yielding a 0.6 wt% platinum-loaded alumina catalyst (hereinafter referred to as a catalyst NO.3). In the catalyst NO.3 thus obtained, the average particle diameter of the platinum particles as measured by direct observation using an electron microscope was 1.27 nm (12.7 Å). Table 3 shows the measurement result of the platinum particle diameter.

[Table 3]

| No. | particle diameter (nm) | No. | particle diameter (nm) | No. | particle diameter (nm) | No. | particle diameter (nm) | No. | particle diameter (nm) |
|---|---|---|---|---|---|---|---|---|---|
| 1 | 1.5 | 11 | 1.0 | 21 | 1.4 | 31 | 1.5 | 41 | 1.0 |
| 2 | 0.9 | 12 | 1.4 | 22 | 1.7 | 32 | 1.5 | 42 | 1.1 |
| 3 | 1.3 | 13 | 1.4 | 23 | 1.2 | 33 | 2.1 | 43 | 1.4 |
| 4 | 1.5 | 14 | 1.8 | 24 | 0.8 | 34 | 1.0 | 44 | 1.2 |
| 5 | 1.3 | 15 | 1.4 | 25 | 1.3 | 35 | 1.1 | 45 | 1.3 |
| 6 | 1.1 | 16 | 1.3 | 26 | 1.5 | 36 | 1.0 | 46 | - |
| 7 | 1.2 | 17 | 0.9 | 27 | 1.1 | 37 | 1.5 | 47 | - |
| 8 | 1.9 | 18 | 1.1 | 28 | 1.2 | 38 | 0.8 | 48 | - |
| 9 | 1.5 | 19 | 1.0 | 29 | 1.0 | 39 | 1.6 | 49 | - |
| 10 | 1.2 | 20 | 0.9 | 30 | 1.1 | 40 | 1.1 | 50 | - |

In Table 3, it is seen that the smallest platinum particle was 0.8 nm (8 Å) in size, and the largest platinum particle was 2.1 nm (21 Å) in size. Also, of the 45 platinum particles measured, 40 (about 89 %) platinum particles had a size in a range of 0.8 to 1.5 nm (8 to 15 Å) , and only 5 platinum particles were larger than 1.5 nm (15 Å) and had a size of 1.6 nm (16 Å) or larger.

[Example 2] (preparation method and particle diameter measurement result of the uniform-type catalyst according to the present invention)

3900 cc of aluminum nitrate aqueous solution with a concentration of 2.67 mol/L was prepared and simultaneously, 3900 cc of 14 % aqueous ammonia solution was prepared. 20 L of pure water was put in a 30-L enamel container, and the container was warmed to 70 °C under stirring. While continuing stirring, a pH swing operation in which 1300 cc of aluminum nitrate aqueous solution was added, followed by stirring for 5 minutes (pH = 2.0), and thereafter, 1300 cc of aqueous ammonia solution was added, followed by stirring for 5 minutes (pH = 7.4) was performed 4 times. An aqueous slurry solution of the obtained aluminum hydroxide was filtered to recover a cake, and subsequently, a washing operation in which the cake was re-dispersed in 20 L of pure water and was filtered again was performed 3 times, obtaining a washed gel.

The washed cake was air dried to adjust the moisture, and then was formed into a rod-like shape having a diameter of 1.6 mm with an extruder. The resultant was dried (120 °C, 3 hours), crushed to about 1 cm in length, and calcined in a muffle furnace (350 °C, 3 hours), thereby yielding an alumina carrier A containing no sulfur. The obtained alumina carrier A had a BET surface area of 290 m$^2$/g and a pore volume of 0.61 cm$^3$/g as measured by mercury porosimetry. Also, the obtained alumina carrier A had an average pore diameter of 9.5 nm (95 Å) and had a sharp pore distribution in which almost all of the pores were concentrated near the average pore diameter. In addition, the volume occupied by pores having a diameter of 7 to 11 nm (70 to 110 Å) was 80 % or more of the total pore volume.

The γ-alumina carrier thus prepared was impregnated with an ammonium sulfate aqueous solution with a concentration of 0.38 mol/L so that the sulfur content after calcination was 0.5 % by weight, and after the solvent was removed with an evaporator, the resultant was dried (120 °C, 3 hours) and calcined (350 °C, 3 hours), thereby yielding an alumina carrier containing sulfur.

The obtained alumina carrier was impregnated with a chloroplatinic acid aqueous solution whose pH was adjusted to 2.0 so that the loading amount of platinum after calcination was 0.6 % by weight. Thereafter, moisture was removed with an evaporator, and the resultant was dried (120 °C, 3 hours) and calcined (350 °C, 3 hours). Then, the alumina carrier was placed in a flow-type hydrogen-reducing apparatus, and hydrogen reduction was carried out at 400 °C for 15 hours in a hydrogen stream, thereby yielding a 0.6 wt% platinum-loaded alumina catalyst (hereinafter referred to as a catalyst NO.4). In the catalyst NO.4 thus obtained, the average particle diameter of the platinum particles as measured by direct observation using an electron microscope was 1.34 nm (13.4 Å). Table 4 shows the measurement result of the platinum particle diameter.

[Table 4]

| No. | particle diameter (nm) | No. | particle diameter (nm) | No. | particle diameter (nm) | No. | particle diameter (nm) | No. | particle diameter (nm) |
|---|---|---|---|---|---|---|---|---|---|
| 1 | 1.3 | 11 | 0.8 | 21 | 1.5 | 31 | 1.5 | 41 | 1.4 |
| 2 | 1.5 | 12 | 1.7 | 22 | 1.3 | 32 | 1.4 | 42 | 1.5 |
| 3 | 1.4 | 13 | 1.5 | 23 | 1.3 | 33 | 1.7 | 43 | 1.6 |
| 4 | 1.9 | 14 | 1.5 | 24 | 1.2 | 34 | 1.1 | 44 | 1.3 |
| 5 | 1.4 | 15 | 1.5 | 25 | 1.4 | 35 | 1.2 | 45 | 1.2 |
| 6 | 1.2 | 16 | 1.4 | 26 | 2.2 | 36 | 0.8 | 46 | 1.4 |
| 7 | 1.3 | 17 | 1.0 | 27 | 1.2 | 37 | 1.0 | 47 | 1.3 |
| 8 | 1.1 | 18 | 1.2 | 28 | 1.1 | 38 | 1.2 | 48 | - |
| 9 | 1.5 | 19 | 1.1 | 29 | 1.1 | 39 | 1.4 | 49 | - |
| 10 | 1.8 | 20 | 1.0 | 30 | 1.2 | 40 | 1.2 | 50 | - |

In Table 4, the smallest platinum particle was 0.8 nm (8 Å) in size, and the largest platinum particle was 2.2 nm (22 Å) in size. Also, of the 47 platinum particles measured, 41 (about 87 %) platinum particles had a size in a range of 0.8 to 1.5 nm (8 to 15 Å), and only 6 platinum particles were larger than 1.5 nm (15 Å) and had a size of 1.6 nm (16 Å) or larger.

[Example 3] (reaction test results of the egg shell-type catalyst and the uniform-type catalyst according to the present invention)

For the egg shell-type platinum-loaded γ-alumina catalyst prepared under the preparation condition according to the present invention shown in Example 1 (catalyst NO.3) and the uniform-type platinum-loaded γ-alumina catalyst prepared under the preparation condition according to the present invention shown in Example 2 (catalyst NO.4), the dehydrogenation reaction test of methylcyclohexane was carried out according to the method and reaction condition similar to the method shown in Comparative Example 4. Table 5 shows the results of the dehydrogenation reaction test together with the average particle diameter of the platinum particles measured based on direct observation of the images taken by the electron microscope and the results of calculating the ratio of the number of platinum particles having a size in a range of 0.8 to 1.5 nm (8 to 15 Å) among the platinum particles measured.

[Table 5]

| catalyst No. | sulfur content (wt%) | plutinum loading amount (wt%) | after 24 hours from initiation of reaction | | | after 300 hours from initiation of reaction | | | plutinum average particle diameter (Å) | | ratio of plutinum particles having diameter in a range of 8-15 Å as measured by direct observation (%) |
|---|---|---|---|---|---|---|---|---|---|---|---|
| | | | MCH conversion rate (%) | toluene selectivity (%) | toluene yield (%) | MCH conversion rate (%) | toluene selectivity (%) | toluene yield (%) | CO-pulse method | direct observation method | |
| 1 | 0 | 0.6 | 98.2 | 99.88 | 98.1 | 94.5 | 99.90 | 94.4 | 5.5 | - | |
| 2 | 0.5 | 0.6 | 98.2 | 99.92 | 98.1 | 97.7 | 99.93 | 97.6 | 6.5 | 16.8 | 45.2 |
| 3 | 0 | 0.6 | 98.4 | 99.91 | 98.3 | 96.2 | 99.90 | 96.1 | - | 12.7 | 88.9 |
| 4 | 0.5 | 0.6 | 98.4 | 99.95 | 98.4 | 98.1 | 99.93 | 98.0 | - | 13.4 | 84.4 |

EP 4 324 559 B1

As seen from the reaction test results of Table 5, as a result of optimizing the condition of catalyst preparation according to the present invention, platinum-loaded alumina catalysts having excellent performance particularly in the catalyst life were obtained. Compared to the egg shell-type platinum-loaded γ-alumina catalyst prepared under the conventional preparation condition (catalyst NO. 1), the egg shell-type platinum-loaded γ-alumina catalyst prepared under the preparation condition according to the present invention (catalyst NO.3) is smaller in the reduction of toluene yield and has a longer life. Also, compared to the uniform-type platinum-loaded γ-alumina catalyst prepared under the conventional preparation condition (catalyst NO.2), the uniform-type platinum-loaded γ-alumina catalyst prepared under the preparation condition according to the present invention (catalyst NO.4) is smaller in the reduction of toluene yield and has a longer life.

Since the reaction test of Table 5 was carried out under the accelerated test condition, the amount of deterioration of toluene yield after about 300 hours appears small. However, the amount of deterioration under the accelerated test condition is remarkably larger than the amount of deterioration under the actual reaction condition in commercialization, and roughly speaking, while the life of the catalyst NO.1 in Table 5 is about one year and the life of the catalyst NO.2 is about two years, the life of the catalyst NO.3 can be expected to be about three years, and the life of the catalyst NO.4 can be expected to be about four years.

In addition, in the catalysts according to the present invention (catalyst NO.3, catalyst NO.4) in which the catalyst life is improved by the preparation methods shown in Example 1 and Example 2, among the all platinum particles for which the platinum particle diameter was measured by direct observation of the observation image taken by the electron microscope, 80 % or more of the platinum particles had a particle diameter in the range of 0.8 to 1.5 nm (8 to 15 Å). Compared to the catalyst NO.2 in which the ratio of the number of particles having a particle diameter in the range of 0.8 to 1.5 nm (8 to 15 Å) was about 45 %, it is seen that in the catalysts according to the present invention, the ratio of the number of platinum particles having a particle diameter in the range of 0.8 to 1.5 nm (8 to 15 Å) is significantly high and the ratio of the platinum particles having a size of 1.6 nm (16 Å) or greater is remarkably decreased.

The reason that the catalyst life of the catalysts prepared by the preparation methods shown in Example 1 and Example 2 according to the present invention (catalyst NO.3, catalyst NO.4) was conspicuously improved as described above is considered to be that the calcination condition when preparing the γ-alumina carrier, the calcination condition after impregnating platinum (and sulfur, if necessary) and drying, and the condition when finally carrying out hydrogen reduction were optimized.

The reason that the life of the conventional catalysts (namely, the dehydrogenation catalysts described in Patent Document 1 and Patent Document 2) was hindered is considered to be that in the conventional preparation method, the calcination condition when preparing the γ-alumina carrier was over 400 °C, the calcination condition after impregnating platinum (and sulfur, if necessary) and drying was similarly at high temperature, and the final hydrogen reduction temperature was high similarly to the calcination conditions when preparing the carrier and after impregnation of platinum (and sulfur, if necessary) and drying. Particularly, it was found to be more preferable that in the preparation of the catalyst, the final hydrogen reduction temperature is set to 400 °C or lower and the calcination which causes thermal history before the hydrogen reduction is carried out at a temperature lower than the final hydrogen reduction condition.

Note that the catalysts according to the present invention cannot be accomplished with the electron microscope technology that could be used for the conventional catalysts (namely, at the time of filing of patent applications related to the dehydrogenation catalysts described in Patent Document 1 and Patent Document 2), and could be accomplished due to evolution of the electron microscope technology thereafter in which the electron microscope technology has been progressed to enable the size of the platinum particles of the catalysts prepared under various catalyst preparation conditions to be accurately measured by direct observation.

INDUSTRIAL APPLICABILITY

The egg shell-type platinum-loaded alumina catalyst and the uniform-type platinum-loaded alumina catalyst according to the present invention can be favorably used in the dehydrogenation reaction of hydrogenated aromatics such as methylcyclohexane used as a hydrogen energy carrier, and can contribute to practical implementation of the hydrogen storage and transportation system according to the organic chemical hydride method. Besides, there is a possibility that they can be widely applied to the existing catalytic reaction processes in which the platinum-loaded alumina catalyst is used. Thus, the present invention has very high industrial applicability.

LIST OF REFERENCE NUMERALS

1 carrier
2 metal loading part

**Claims**

1. A platinum-loaded alumina catalyst, comprising:

   an alumina carrier (1); and
   platinum loaded on the alumina carrier (1),
   wherein the alumina carrier (1) comprises a γ-alumina carrier that has a BET surface area of 200 m$^2$/g or more, a pore volume of 0.50 cm$^3$/g or more as measured by mercury porosimetry, and an average pore diameter in a range of 6.0 to 15.0 nm (60 to 150 Å), with pores having a pore diameter in a range of $\pm$ 3.0 nm ($\pm$30 Å) from the average pore diameter occupying 60 % or more of a total pore volume,
   **characterized in that** particles of the platinum are loaded on the γ-alumina carrier in a range of 0.1 to 1.5 % by weight calculated as elemental platinum (Pt), and
   70 % or more of the particles of the platinum have a size of 0.8 to 1.5 nm (8 to 15 Å) by direct observation using a transmission electron microscope.

2. The platinum-loaded alumina catalyst according to claim 1, wherein the γ-alumina carrier contains sulfur or a sulfur compound in a range of 0.5 to 1.2 % by weight calculated as elemental sulfur (S).

3. The platinum-loaded alumina catalyst according to claim 1 or 2, wherein the γ-alumina carrier has alkali metals loaded thereon in a range of 0.5 to 1.5 % by weight, and
   the alkali metals are sodium and potassium.

4. A method of producing the platinum-loaded alumina catalyst according to claim 1,

   wherein in a preparation of the γ-alumina carrier,
   an alkaline aqueous solution is added to an acidic aqueous solution containing aluminum, and a boehmite obtained as aluminum hydroxide is dried and thereafter is calcined at a temperature in a range of 250 to 400 °C for a time period in a range of 1 to 12 hours.

5. The method of producing the platinum-loaded alumina catalyst according to claim 4, wherein, by using a chloroplatinic acid aqueous solution as a platinum reagent aqueous solution, the γ-alumina carrier after the calcination is impregnated with the platinum such that a content thereof is in a range of 0.5 to 1.5 % by weight calculated as elemental platinum, and a resultant is dried and thereafter is calcined at a temperature in a range of 250 to 400 °C.

6. The method of producing the platinum-loaded alumina catalyst according to claim 5, wherein the γ-alumina carrier that has been impregnated with the platinum, dried, and thereafter calcined is subjected to hydrogen reduction, and
   a temperature of the hydrogen reduction is higher than the temperature for calcining the boehmite after drying, is higher than the temperature for calcining the γ-alumina carrier impregnated with the platinum and dried, and is in a range of 300 to 450 °C.

7. The method of producing the platinum-loaded alumina catalyst according to claim 6, wherein a time period of the hydrogen reduction is in a range of 1 to 15 hours.

8. The method of producing the platinum-loaded alumina catalyst according to any one of claims 4 to 7, wherein the γ-alumina carrier contains sulfur or a sulfur compound in a range of 0.5 to 1.2 % by weight calculated as elemental sulfur (S).

9. The method of producing the platinum-loaded alumina catalyst according to any one of claims 4 to 8, wherein the γ-alumina carrier after the calcination is impregnated with ammonium sulfate aqueous solution and thereafter is calcined at a temperature in a range of 250 to 400 °C for a time period in range of 1 to 12 hours.

10. The method of producing the platinum-loaded alumina catalyst according to claim 5, wherein the γ-alumina carrier containing no sulfur or the γ-alumina carrier containing sulfur is impregnated with platinum, is dried, and thereafter is calcined to generate a platinum-loaded γ-alumina carrier,

    the platinum-loaded γ-alumina carrier is impregnated with alkali metals such that the alkali metals are contained in a range of 0.5 to 1.5 % by weight, is dried, and thereafter is subjected to hydrogen reduction without being calcined, and

a temperature of the hydrogen reduction is higher than the temperature for calcining the boehmite after drying, is higher than the temperature for calcining the γ-alumina carrier impregnated with the platinum and dried, and is in a range of 300 to 450 °C.

11. The method of producing the platinum-loaded alumina catalyst according to claim 10, wherein the alkali metals are sodium and potassium.

12. The method of producing the platinum-loaded alumina catalyst according to claim 10 or 11, wherein a time period of the hydrogen reduction is in a range of 1 to 15 hours.

13. A method of dehydrogenating a hydrogenated aromatic, comprising dehydrogenating a hydrogenated aromatic by using the platinum-loaded alumina catalyst according to any one of claims 1 to 3.

14. The method of dehydrogenating a hydrogenated aromatic according to claim 13, wherein the hydrogenated aromatic is one member or a mixture of two or more members selected from the group consisting of a hydride of monocyclic aromatic, a hydride of bicyclic aromatic, and a hydride of compound having 3 or more aromatic rings.

15. The method of dehydrogenating a hydrogenated aromatic according to claim 13, wherein the hydrogenated aromatic is one member or a mixture of two or more members selected from the group consisting of methylcyclohexane, cyclohexane, trimethylcyclohexane and decalin.

**Patentansprüche**

1. Mit Platin beladener Aluminiumoxidkatalysator, umfassend:

einen Aluminiumoxidträger (1); und
auf den Aluminiumoxidträger (1) geladenes Platin,
wobei der Aluminiumoxidträger (1) einen γ-Aluminiumoxidträger umfasst, der eine BET-Oberfläche von 200 $m^2$/g oder mehr, ein Porenvolumen von 0,50 $cm^3$/g oder mehr, gemessen durch Quecksilberporosimetrie, und einen durchschnittlichen Porendurchmesser in einem Bereich von 6,0 bis 15,0 nm (60 bis 150 Å) aufweist, wobei Poren mit einem Porendurchmesser in einem Bereich von ± 3,0 nm (±30 Å) von dem durchschnittlichen Porendurchmesser 60 % oder mehr des Gesamtporenvolumens einnehmen,
**dadurch gekennzeichnet, dass** Partikel des Platins in einem Bereich von 0,1 bis 1,5 Gew.-%, berechnet als elementares Platin (Pt), auf den γ-Aluminiumoxidträger geladen sind, und
70 % oder mehr der Platinpartikel eine Größe von 0,8 bis 1,5 nm (8 bis 15 Å) durch direkte Beobachtung unter Verwendung eines Transmissionselektronenmikroskops aufweisen.

2. Mit Platin beladener Aluminiumoxidkatalysator nach Anspruch 1, wobei der γ-Aluminiumoxidträger Schwefel oder eine Schwefelverbindung in einem Bereich von 0,5 bis 1,2 Gew.-%, berechnet als elementarer Schwefel (S), enthält.

3. Mit Platin beladener Aluminiumoxidkatalysator nach Anspruch 1 oder 2, wobei der γ-Aluminiumoxidträger mit Alkalimetallen in einem Bereich von 0,5 bis 1,5 Gew.-% beladen ist, und
die Alkalimetalle Natrium und Kalium sind.

4. Verfahren zur Herstellung des mit Platin beladenen Aluminiumoxidkatalysators nach Anspruch 1,

wobei bei der Herstellung des γ-Aluminiumoxidträgers,
eine alkalische wässrige Lösung zu einer sauren wässrigen Lösung hinzugefügt wird, die Aluminium enthält, und
ein als Aluminiumhydroxid erhaltenes Böhmit getrocknet wird und danach bei einer Temperatur in einem Bereich von 250 bis 400 °C für einen Zeitraum in einem Bereich von 1 bis 12 Stunden kalziniert wird.

5. Verfahren zur Herstellung des mit Platin beladenen Aluminiumoxidkatalysators nach Anspruch 4, wobei unter Verwendung einer wässrigen Chlorplatinatsäurelösung als eine wässrige Platinreagenzlösung der γ-Aluminiumoxidträger nach dem Kalzinieren mit dem Platin imprägniert wird, so dass dessen Gehalt in einem Bereich von 0,5 bis 1,5 Gew.-%, berechnet als elementares Platin, liegt, und das resultierende Produkt getrocknet wird und danach bei einer Temperatur in einem Bereich von 250 bis 400 °C kalziniert wird.

**6.** Verfahren zur Herstellung des mit Platin beladenen Aluminiumoxidkatalysators nach Anspruch 5, wobei der mit Platin imprägnierte, getrocknete und danach kalzinierte γ-Aluminiumoxidträger einer Wasserstoffreduktion unterzogen wird, und

eine Temperatur der Wasserstoffreduktion höher ist als die Temperatur zum Kalzinieren des Böhmits nach dem Trocknen, höher ist als die Temperatur zum Kalzinieren des mit Platin imprägnierten und getrockneten γ-Aluminiumoxidträgers und in einem Bereich von 300 bis 450 °C liegt.

**7.** Verfahren zur Herstellung des mit Platin beladenen Aluminiumoxidkatalysators nach Anspruch 6, wobei eine Dauer der Wasserstoffreduktion in einem Bereich von 1 bis 15 Stunden liegt.

**8.** Verfahren zur Herstellung des mit Platin beladenen Aluminiumoxidkatalysators nach einem der Ansprüche 4 bis 7, wobei der γ-Aluminiumoxidträger Schwefel oder eine Schwefelverbindung in einem Bereich von 0,5 bis 1,2 Gew.-%, berechnet als elementarer Schwefel (S), enthält.

**9.** Verfahren zur Herstellung des mit Platin beladenen Aluminiumoxidkatalysators nach einem der Ansprüche 4 bis 8, wobei der γ-Aluminiumoxidträger nach dem Kalzinieren mit einer wässrigen Ammoniumsulfatlösung imprägniert wird und danach bei einer Temperatur in einem Bereich von 250 bis 400 °C für einen Zeitraum im Bereich von 1 bis 12 Stunden kalziniert wird.

**10.** Verfahren zur Herstellung des mit Platin beladenen Aluminiumoxidkatalysators nach Anspruch 5, wobei der γ-Aluminiumoxidträger, der keinen Schwefel enthält, oder der γ-Aluminiumoxidträger, der Schwefel enthält, mit Platin imprägniert wird, getrocknet wird und danach kalziniert wird, um einen mit Platin beladenen γ-Aluminiumoxidträger zu erzeugen,

der mit Platin beladene γ-Aluminiumoxidträger mit Alkalimetallen imprägniert wird, sodass die Alkalimetalle in einem Bereich von 0,5 bis 1,5 Gew.-% enthalten sind, getrocknet wird und danach einer Wasserstoffreduktion unterzogen wird, ohne kalziniert zu werden, und

eine Temperatur der Wasserstoffreduktion höher ist als die Temperatur zum Kalzinieren des Böhmits nach dem Trocknen, höher ist als die Temperatur zum Kalzinieren des mit Platin imprägnierten und getrockneten γ-Aluminiumoxidträgers und in einem Bereich von 300 bis 450 °C liegt.

**11.** Verfahren zur Herstellung des mit Platin beladenen Aluminiumoxidkatalysators nach Anspruch 10, wobei die Alkalimetalle Natrium und Kalium sind.

**12.** Verfahren zur Herstellung des mit Platin beladenen Aluminiumoxidkatalysators nach Anspruch 10 oder 11, wobei eine Dauer der Wasserstoffreduktion in einem Bereich von 1 bis 15 Stunden liegt.

**13.** Verfahren zum Dehydrieren eines hydrierten Aromaten, umfassend das Dehydrieren eines hydrierten Aromaten unter Verwendung des mit Platin beladenen Aluminiumoxidkatalysators nach einem der Ansprüche 1 bis 3.

**14.** Verfahren zum Dehydrieren eines hydrierten Aromaten nach Anspruch 13, wobei der hydrierte Aromat ein Mitglied oder eine Mischung aus zwei oder mehr Mitgliedern ist, ausgewählt aus der Gruppe bestehend aus einem Hydrid eines monocyclischen Aromaten, einem Hydrid eines bicyclischen Aromaten und einem Hydrid einer Verbindung mit 3 oder mehr aromatischen Ringen.

**15.** Verfahren zum Dehydrieren eines hydrierten Aromaten nach Anspruch 13, wobei der hydrierte Aromat ein Mitglied oder eine Mischung aus zwei oder mehr Mitgliedern ist, ausgewählt aus der Gruppe bestehend aus Methylcyclohexan, Cyclohexan, Trimethylcyclohexan und Decalin.

**Revendications**

**1.** Catalyseur d'alumine chargé de platine, comprenant :

un support d'alumine (1) ; et
le platine chargé sur le support d'alumine (1),
dans lequel le support d'alumine (1) comprend un support d'alumine γ qui présente une surface BET de 200 m$^2$/g ou plus, un volume poreux de 0,50 cm$^3$/g ou plus tel que mesuré par porosimétrie au mercure, et un diamètre poreux moyen compris dans une plage de 6,0 nm à 15,0 nm (60 Å à 150 Å), avec des pores présentant un diamètre poreux compris dans une plage de ± 3,0 nm (± 30 Å) par rapport au diamètre poreux moyen occupant 60 % ou plus d'un volume poreux total,

**caractérisé en ce que** des particules du platine sont chargées sur le support d'alumine γ dans une plage comprise entre 0,1 % et 1,5 % en poids calculé comme platine élémentaire (Pt), et

70 % ou plus des particules du platine présentent une taille comprise entre 0,8 nm et 1,5 nm (8 Å à 15 Å) par observation directe en utilisant un microscope électronique en transmission.

2. Catalyseur d'alumine chargé de platine selon la revendication 1, dans lequel le support d'alumine y contient du soufre ou un composé de soufre dans une plage comprise entre 0,5 % et 1,2 % en poids calculé comme soufre élémentaire (S).

3. Catalyseur d'alumine chargé de platine selon la revendication 1 ou la revendication 2, dans lequel le support d'alumine y présente des métaux alcalins chargés sur celui-ci dans une plage comprise entre 0,5 % et 1,5 % en poids, et

les métaux alcalins sont du sodium et du potassium.

4. Procédé de production du catalyseur d'alumine chargé de platine selon la revendication 1,

dans lequel dans une préparation du support d'alumine γ,
une solution aqueuse alcaline est ajoutée à une solution aqueuse acide contenant de l'aluminium, et une boehmite obtenue comme hydroxyde d'aluminium est séchée et par la suite calcinée à une température comprise dans une plage de 250 °C à 400 °C pendant une période de temps comprise dans une plage de 1 heure à 12 heures.

5. Procédé de production du catalyseur d'alumine chargé de platine selon la revendication 4, dans lequel, en utilisant une solution aqueuse d'acide chloroplatinique comme une solution aqueuse de réactif de platine, le support d'alumine y après la calcination est imprégné du platine de sorte qu'une teneur de celui-ci soit comprise dans une plage de 0,5 % à 1,5 % en poids calculée comme platine élémentaire, et une résultante est séchée et par la suite calcinée à une température comprise dans une plage de 250 °C à 400 °C.

6. Procédé de production du catalyseur d'alumine chargé de platine selon la revendication 5, dans lequel le support d'alumine γ qui a été imprégné du platine, séché, et par la suite calciné est soumis à une réduction par l'hydrogène, et une température de la réduction par l'hydrogène est supérieure à la température de calcination de la boehmite après séchage, est supérieure à la température de calcination du support d'alumine γ imprégné du platine et séché, et est comprise dans une plage de 300 °C à 450 °C.

7. Procédé de production du catalyseur d'alumine chargé de platine selon la revendication 6, dans lequel une période de temps de la réduction par l'hydrogène est comprise dans une plage de 1 heure à 15 heures.

8. Procédé de production du catalyseur d'alumine chargé de platine selon l'une quelconque des revendications 4 à 7, dans lequel le support d'alumine y contient du soufre ou un composé de soufre dans une plage comprise entre 0,5 % et 1,2 % en poids calculé comme soufre élémentaire (S).

9. Procédé de production du catalyseur d'alumine chargé de platine selon l'une quelconque des revendications 4 à 8, dans lequel le support d'alumine y après la calcination est imprégné d'une solution aqueuse de sulfate d'ammonium et par la suite est calciné à une température comprise dans une plage de 250 °C à 400 °C pendant une période de temps comprise dans une plage de 1 heure à 12 heures.

10. Procédé de production du catalyseur d'alumine chargé de platine selon la revendication 5, dans lequel le support d'alumine y ne contenant pas de soufre ou le support d'alumine y contenant du soufre est imprégné de platine, est séché, et par la suite est calciné pour générer un support d'alumine γ chargé de platine,

le support d'alumine γ chargé de platine est imprégné de métaux alcalins de sorte que les métaux alcalins soient contenus dans une plage comprise entre 0,5 % et 1,5 % en poids, est séché, et par la suite est soumis à une réduction par l'hydrogène sans être calciné, et
une température de la réduction par l'hydrogène est supérieure à la température de calcination de la boehmite après séchage, est supérieure à la température de calcination du support d'alumine γ imprégné du platine et séché, et est comprise dans une plage de 300 °C à 450 °C.

11. Procédé de production du catalyseur d'alumine chargé de platine selon la revendication 10, dans lequel les métaux alcalins sont du sodium et du potassium.

12. Procédé de production du catalyseur d'alumine chargé de platine selon la revendication 10 ou la revendication 11, dans lequel une période de temps de la réduction par l'hydrogène est comprise dans une plage de 1 heure à 15 heures.

13. Procédé de déshydrogénation d'un aromatique hydrogéné, comprenant la déshydrogénation d'un aromatique hydrogéné en utilisant le catalyseur d'alumine chargé de platine selon l'une quelconque des revendications 1 à 3.

14. Procédé de déshydrogénation d'un aromatique hydrogéné selon la revendication 13, dans lequel l'aromatique hydrogéné est un élément ou un mélange de deux ou plusieurs éléments choisis dans le groupe consistant en un hydrure d'aromatique monocyclique, un hydrure d'aromatique bicyclique et un hydrure de composé présentant 3 ou plus cycles aromatiques.

15. Procédé de déshydrogénation d'un aromatique hydrogéné selon la revendication 13, dans lequel l'aromatique hydrogéné est un élément ou un mélange de deux ou plusieurs éléments choisis dans le groupe consistant en méthylcyclohexane, cyclohexane, triméthylcyclohexane et décaline.

**Fig.1**

HD-2000  200kV  1.8 million times

(A)

JEM-ARM200  200kV  2 million times

(B)

EP 4 324 559 B1

# *Fig.2*

(A)

(B)

## Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4652695 B

- JP 4142733 B

**Non-patent literature cited in the description**

- **OKADA YOSHIMI**. *Energy/Natural Resources*, 2018, vol. 33 (3), 168

- **OKADA YOSHIMI**. *Bulletin of The High Pressure Gas Safety Institute of TOKYO*, August 2019
- *Hydrogen Basic Strategy*, December 2017